# FASCICULE DE BREVET EUROPEEN

(11) **EP 2 735 298 B1**
(45) Date de publication et mention de la délivrance du brevet: **29.03.2017**
(21) Numéro de dépôt: 13193488.7
(22) Date de dépôt: 19.11.2013
(51) Int. Cl.: A61H 7/00, A61N 5/06, A61N 1/30, A61H 23/02, A61N 1/32, A61N 1/04

(54) **Appareil de massage avec tête de massage équipee d'embouts se déplacant vers le centre de la tête**
Massagegerät mit einem Massagekopf, der mit Tüllen ausgestattet ist, die sich zur Mitte des Kopfes verschieben
Massage apparatus with massage head provided with nozzles moving towards the centre of the head

(30) Priorité: 22.11.2012 FR 1261109
(43) Date de publication de la demande: 28.05.2014
(73) Titulaire: SEB S.A., 69130 Ecully (FR)
(72) Inventeur: Giraud, Camille, 69001 LYON (FR); Mandica, Franck, 69340 FRANCHEVILLE (FR)
(74) Mandataire: Guéry-Jacques, Géraldine

(56) Documents cités:
- WO-A1-2010/034802
- WO-A1-2011/105224
- WO-A2-2005/091748
- DE-U1- 29 513 016
- US-A1- 2005 020 946

## Description

La présente invention concerne le domaine des appareils de traitement de la peau, notamment celle du visage. L'appareil selon l'invention permet, pour le moins, le massage de la peau afin de lui donner de la tonicité. L'appareil de massage selon l'invention trouvera son application chez personnes désireuses de soigner leur esthétique en remodelant, raffermissant et rajeunissant leur peau, notamment celle du visage.

Les appareils de massage de la peau se composent généralement d'un corps muni de moyens de motorisation et d'une tête de massage qui comprend des éléments de massage configurés pour être activés sous l'action des moyens de motorisation, par le biais d'un mécanisme de transmission. Parmi l'art antérieur existant dans ce domaine, il est connu les brevets US 3 499 439 A et US 1 931 849 A qui divulguent chacun un appareil de massage dont la tête de massage comprend une surface d'application et au moins trois embouts de massage qui s'étendent vers l'extérieur de la surface d'application et se déplacent dans un plan parallèle à cette surface d'application pour réaliser un pincé de la peau, dit « pincé Jacquet », pour masser le visage par pétrissages et petits pincements minutieux en tous sens. Dans le brevet US 3 499 439 A, les embouts sont agencés sur des roues dentées qui engrènent entre elles pour permettre leur rotation en sens inverse les unes par rapport aux autres, les embouts tournant respectivement en cercle avec ces roues dentées, ce qui permet le rapprochement des embouts et ainsi, le pincement de la peau sur laquelle sont appliqués ces embouts. Il en est de même dans le brevet US 1 931 849 A, selon lequel les embouts sont inclinés par rapport à la surface d'application et sont montés au niveau de la portion centrale, en liaison rotule, leur extrémité étant également en liaison rotule sur des roues dentées qui engrènent entre elles et tournent en sens inverse les unes par rapport aux autres. Ainsi, les extrémités opposées des embouts en contact avec la peau définissent des cercles respectifs et se rapprochent pour pincer la peau. US 2005020946 décrit un appareil de massage comprenant un corps qui comporte des moyens de motorisation et une tête de massage.

L'objet de l'invention est de concevoir un appareil de massage permettant d'optimiser le travail de la peau par pincement de celle-ci en vue de travailler la peau et les muscles du visage de façon plus complète, agréable, efficace, par rapport aux appareils de massage de l'art antérieur.

A cet effet, l'invention concerne un appareil de massage comprenant un corps qui comporte des moyens de motorisation et, une tête de massage. Des moyens d'assemblage sont agencés entre le corps et la tête de massage, diverses conceptions de moyens d'assemblage étant envisageables, comme cela sera précisé ultérieurement. La tête de massage comporte une surface d'application et au moins trois embouts de massage qui s'étendent plus ou moins perpendiculairement vers l'extérieur de la surface d'application. Lors de l'utilisation de l'appareil de massage, la surface d'application est positionnée plus ou moins parallèlement à proximité de la surface de peau de l'utilisateur, tandis que les embouts sont en contact sur la peau. Selon l'invention, les embouts de massage sont définis sur la surface d'application selon des cercles virtuels concentriques de centre C. En outre, selon l'invention, la tête de massage comprend un mécanisme de transmission qui est configuré pour être entraîné par les moyens de motorisation et pour rapprocher les embouts de massage et/ou, inversement, pour écarter les embouts de massage, en translatant lesdits embouts de massage dans un sens et/ou dans l'autre selon des trajectoires qui se rejoignent au centre C de ces cercles virtuels. Ainsi, les au moins trois embouts de massage effectuent des mouvements de translation alternatif en va-et-vient relativement à un point défini par le centre C des cercles virtuels, ce qui permet d'obtenir un pincé convenable de la peau et, ainsi, d'optimiser le travail de la peau avec l'appareil de massage. Selon l'invention, la translation des embouts de massage peut être rectiligne dirigée vers le centre C, de préférence de manière radiale, ou curviligne dirigée vers le centre C, en fonction des diverses variantes de conception du mécanisme de transmission qui seront précisées ensuite. Ainsi, l'appareil de massage permet de réaliser un pincé dit simple ou un pincé dit tournant ou oscillant.

Les moyens de motorisation sur l'appareil de massage pourront être de diverses conceptions, en fonction des mouvements à transmettre aux éléments de la tête de massage, voire de l'actionnement d'autres système et/ou dispositifs se trouvant sur l'appareil de massage. Les moyens de motorisation comprendront notamment un ou plusieurs moteurs et des moyens d'engrenage ou d'entraînement pour la transmission de mouvements de rotation, d'oscillation et de translation.

Les moyens d'assemblage entre la tête de massage et le corps pour être de diverses conception, allant d'une tête de massage réalisée en une seule pièce avec le corps, jusqu'au système d'assemblage amovible entre la tête de massage et le corps.

Selon un premier mode de réalisation de l'appareil de massage objet de l'invention, celui-ci comprend trois embouts de massage. En outre, le mécanisme de transmission est configuré pour translater concomitamment les embouts de massage dans un sens puis, inversement, dans l'autre sens, les trajectoires des embouts de massage étant des translations radiales de centre C. Cela permet de réaliser un pincé simple au moyen de trois embouts de massage.

De manière préférentielle et non limitative, selon ce premier mode de réalisation de l'appareil de massage, le mécanisme de transmission comprend un système de guidage fixe sur la tête de massage, trois liaisons glissières étant agencées respectivement entre les trois embouts de massage et le système de guidage et configurées pour translater radialement vis-à-vis du centre C lesdits embouts de massage. Par ailleurs, le mécanisme de transmission comprend un système de retour des embouts de massage qui est configuré pour exercer une poussée contre les embouts de massage visant à écarter lesdits embouts de massage en translation sur le système de guidage. En outre, le mécanisme de transmission comprend un système de rapprochement des embouts de massage qui est configuré pour être entraîné en rotation par les moyens de motorisation selon un axe perpendiculaire à la surface d'application et passant par le centre C. Ce système de rapprochement des embouts de massage est également configuré, d'une part, pour pousser contre les embouts de massage et les rapprocher en translation sur le système de guidage, sur des premières portions angulaires durant la rotation dudit système de rapprochement des embouts de massage et, d'autre part, pour autoriser le système de retour des embouts de massage à écarter les embouts de massage en translation sur le système de guidage, sur des secondes portions angulaires durant la rotation dudit système de rapprochement des embouts de massage.

Selon un second mode de réalisation de l'appareil de massage objet de l'invention, celui-ci comprend trois embouts de massage, le mécanisme de transmission étant configuré pour translater concomitamment les embouts de massage dans un sens puis, inversement, dans l'autre sens, les trajectoires des embouts de massage étant des translations curvilignes dirigées vers le centre C. Cela permet de réaliser un pincé tournant au moyen de trois embouts de massage.

De manière préférentielle et non limitative, selon ce second mode de réalisation de l'appareil de massage, le mécanisme de transmission comprend un système de guidage qui est configuré pour être entraîné en rotation par les moyens de motorisation selon un axe perpendiculaire à la surface d'application et passant par le centre C, trois liaisons glissières étant agencées respectivement entre les trois embouts de massage et le système de guidage et, configurées pour translater radialement vis-à-vis du centre C lesdits embouts de massage. Par ailleurs, le mécanisme de transmission comprend un système de retour des embouts de massage qui est configuré pour exercer une poussée contre les embouts de massage visant à écarter lesdits embouts de massage en translation sur le système de guidage. En outre, le mécanisme de transmission comprend un système de rapprochement des embouts de massage fixe sur la tête de massage et configuré, d'une part, pour pousser contre les embouts de massage et les rapprocher en translation sur le système de guidage, sur des premières portions angulaires durant la rotation dudit système de guidage et, d'autre part, pour autoriser le système de retour des embouts de massage à écarter les embouts de massage en translation sur le système de guidage, sur des secondes portions angulaires durant la rotation dudit système de guidage.

Selon un troisième mode de réalisation de l'appareil de massage objet de l'invention, celui-ci lequel comprend au moins trois embouts de massage, le mécanisme de transmission étant configuré pour translater avec un décalage régulier les embouts de massage dans un sens puis, inversement, dans l'autre sens, les trajectoires des embouts de massage étant des translations radiales de centre C. Cela permet de réaliser un pincé simple mais avec un décalage entre les embouts de massage.

De manière préférentielle et non limitative, selon ce troisième mode de réalisation de l'appareil de massage, le mécanisme de transmission comprend un système de guidage fixe sur la tête de massage, trois liaisons glissières étant agencées respectivement entre les trois embouts de massage et le système de guidage et, configurées pour translater radialement vis-à-vis du centre C lesdits embouts de massage. Par ailleurs, le mécanisme de transmission comprend un système de retour des embouts de massage qui est configuré pour exercer une poussée contre les embouts de massage visant à écarter lesdits embouts de massage en translation sur le système de guidage. En outre, un système de rapprochement des embouts de massage est configuré pour être entraîné en rotation par les moyens de motorisation selon un axe perpendiculaire à la surface d'application et passant par le centre C. Ce système de rapprochement des embouts de massage est également configuré pour pousser progressivement contre un des trois embouts de massage et le rapprocher en translation sur le système de guidage et concomitamment, pour autoriser le système de retour des embouts de massage à écarter progressivement l'embout de massage précédant en translation sur le système de guidage, durant la rotation du système de rapprochement des embouts de massage. Et ainsi de suite successivement sur les trois embouts durant la rotation dudit système de rapprochement des embouts de massage. Une conception similaire peut être envisagée avec quatre embouts de massage.

Selon un quatrième mode de réalisation de l'appareil de massage objet de l'invention, celui-ci comprend quatre embouts de massage, le mécanisme de transmission étant configuré pour translater concomitamment les embouts de massage dans un sens puis, inversement, dans l'autre sens, les trajectoires des embouts de massage étant des translations radiales de centre C. Cela permet de réaliser un pincé simple au moyens de quatre embouts de massage.

De manière préférentielle et non limitative, selon ce quatrième mode de réalisation de l'appareil de massage, le mécanisme de transmission comprend un système de guidage fixe sur la tête de massage, quatre liaisons glissières étant agencées respectivement entre les quatre embouts de massage et le système de guidage et configurées pour translater radialement vis-à-vis du centre C lesdits embouts de massage. Par ailleurs, le mécanisme de transmission comprend un système de retour des embouts de massage configuré pour exercer une poussée contre les embouts de massage visant à écarter lesdits embouts de massage en translation sur le système de guidage. En outre, le mécanisme comprend un système de rapprochement des embouts de massage configuré pour être entraîné en rotation par les moyens de motorisation selon un axe perpendiculaire à la surface d'application et passant par le centre C. Ce système de rapprochement des embouts de massage est également configuré, d'une part, pour pousser contre les embouts de massage et les rapprocher en translation sur le système de guidage, sur des premières portions angulaires durant la rotation du système de rapprochement des embouts de massage et, d'autre part, pour autoriser le système de retour des embouts de massage à écarter les embouts de massage en translation sur le système de guidage, sur des secondes portions angulaires durant la rotation du système de rapprochement des embouts de massage.

Selon un cinquième mode de réalisation de l'appareil de massage objet de l'invention, celui-ci comprend quatre embouts de massage, le mécanisme de transmission étant configuré pour translater concomitamment les embouts de massage dans un sens puis, inversement, dans l'autre sens, les trajectoires des embouts de massage étant des translations curvilignes dirigées vers le centre C. Cela permet de réaliser un pincé tournant au moyen de quatre embouts de massage.

De manière préférentielle et non limitative, selon ce cinquième mode de réalisation de l'appareil de massage, le mécanisme de transmission comprend un système de guidage configuré pour être entraîné en rotation par les moyens de motorisation selon un axe perpendiculaire à la surface d'application et passant par le centre C, quatre liaisons glissières étant agencées respectivement entre les quatre embouts de massage et le système de guidage et configurées pour translater radialement vis-à-vis du centre C, lesdits embouts de massage. Par ailleurs, le mécanisme de transmission comprend un système de retour des embouts de massage configuré pour exercer une poussée contre les embouts de massage visant à écarter lesdits embouts de massage en translation sur le système de guidage. En outre, le mécanisme de transmission comprend un système de rapprochement des embouts de massage fixe sur la tête de massage et configuré, d'une part, pour pousser contre les embouts de massage et les rapprocher en translation sur le système de guidage, sur des premières portions angulaires durant la rotation dudit système de guidage et, d'autre part, pour autoriser le système de retour des embouts de massage à écarter les embouts de massage en translation sur le système de guidage, sur des secondes portions angulaires durant la rotation dudit système de guidage.

Selon un sixième mode de réalisation de l'appareil de massage objet de l'invention, celui-ci comprend quatre embouts de massage, le mécanisme de transmission étant configuré pour translater concomitamment dans un sens, les deux premiers embouts de massage disposés en vis-à-vis et dans l'autre sens, les deux seconds embouts de massage disposés en vis-à-vis, puis inversement, les trajectoires des embouts de massage étant des translations radiales de centre C. Cela permet de réaliser un pincé simple alternatif au moyen de quatre embouts de massage.

De manière préférentielle et non limitative, selon ce sixième mode de réalisation de l'appareil de massage, le mécanisme de transmission comprend un système de guidage fixe sur la tête de massage, quatre liaisons glissières étant agencées respectivement entre les quatre embouts de massage et le système de guidage et configurées pour translater radialement vis-à-vis du centre C lesdits embouts de massage. Par ailleurs, le mécanisme de transmission comprend un système de retour des embouts de massage configuré pour exercer une poussée contre les embouts de massage visant à écarter lesdits embouts de massage en translation sur le système de guidage. En outre, le mécanisme de transmission comprend un système de rapprochement des embouts de massage configuré pour être entraîné en rotation par les moyens de motorisation selon un axe perpendiculaire à la surface d'application et passant par le centre C. Ce système de rapprochement des embouts de massage est également configuré pour pousser contre les deux premiers embouts de massage en vis-à-vis et les rapprocher en translation sur le système de guidage et, concomitamment, pour autoriser le système de retour des embouts de massage à écarter en translation sur le système de guidage les deux seconds embouts de massage en vis-à-vis, sur des premières portions angulaires durant la rotation du système de rapprochement des embouts de massage et, inversement sur des secondes portions angulaires durant la rotation du système de rapprochement des embouts de massage.

Selon les divers modes de réalisation préférentiels précités de l'appareil de massage, le système de retour des embouts de massage et le système de rapprochement des embouts de massage sur le mécanisme de transmission peuvent être indépendants ou, au contraire, mis en oeuvre par les mêmes éléments. En effet, le système de rapprochement des embouts de massage peut être configuré pour exercer la fonction précitée du système de retour des embouts de massage.

Selon ces divers modes de réalisation préférentiels de l'appareil de massage objet de l'invention, le système de rapprochement des embouts de massage comprend des moyens d'amortissement des efforts exercés par les embouts de massage sur la peau. Cela évite un pincement trop important de la peau et, ainsi, des sensations désagréables sur la peau durant l'utilisation de l'appareil de massage.

Selon l'appareil de massage objet de l'invention, celui-ci comprend une couronne qui s'étend perpendiculairement à la surface d'application et, un second mécanisme de transmission entraîné par les moyens de motorisation, configuré pour entraîner la couronne avec un mouvement oscillatoire ou de rotation autour d'un axe perpendiculaire à la surface d'application et passant par le centre C. Cette couronne est dans une matière souple configurée pour bien agripper la peau. Cette couronne permet de travailler la peau de manière complémentaire au pincement en sorte de réaliser un travail complexe de la peau efficace et bien perçu par l'utilisateur.

Selon l'appareil de massage objet de l'invention, celui-ci comprend un système d'émission d'ondes au niveau de la surface d'application. Ces ondes seront par exemple des ondes electromagnétiques, particulièrement lumineuses, visibles ou dans le domaine de l'infrarouge, ou peuvent être par exemple sonores par exemple des ultrasons. Cela permet également de travailler de manière complémentaire, la peau de l'utilisateur. Dans un exemple envisagé de réalisation, ces ondes sont lumineuses de couleur rouge ou orange.

Selon l'appareil de massage objet de l'invention, les embouts de massage sont dans une matière et de forme configurées pour accrocher et travailler la peau sans douleur.

Selon l'appareil de massage objet de l'invention, celui-ci comprend un système de distribution d'un produit cosmétique configuré pour distribuer directement ou indirectement ledit produit cosmétique sur les embouts de massage et/ou directement sur la peau de l'utilisateur. Selon la configuration du système de distribution, la distribution du produit cosmétique pourra être réalisée de manière naturelle, manuelle et/ou automatique. Cela permet de réaliser également un traitement complémentaire à celui réalisé par le pincement de la peau.

Selon l'appareil de massage objet de l'invention, celui-ci comprend une peau souple qui recouvre les embouts de massage. Cette peau souple permet notamment d'éviter que les embouts de massage ne paraissent trop agressifs et d'agripper encore mieux la peau grâce à un contact surfacique sur la peau.

Selon l'appareil de massage objet de l'invention, celui-ci comprend un système vibratoire configuré pour faire vibrer la tête de massage voire seulement les embouts de massage. Cela permet également de travailler la peau au moyen de petites pressions sur la peau, en complément du pincement.

Selon l'appareil de massage objet de l'invention, les moyens d'assemblage entre le corps et la tête de massage sont configurés pour connecter de manière amovible la tête de massage sur le corps. Cela permet de remplacer rapidement la tête de massage par une autre tête de massage identique ou différente.

Selon l'appareil de massage objet de l'invention, celui-ci comprend un dispositif de traitement ionophorèse transcutanée qui est configuré pour transmettre sur la peau lors de l'application dudit appareil de massage, un courant permettant d'augmenter et/ou d'accélerer la pénétration d'un produit cosmétique qui peut être soit distribué par le système de distribution de produit cosmétique décrit ci-dessus, soit appliqué directement sur la peau par l'utilisateur. L'ionophorèse est un traitement qui a été développée initialement pour l'application dans la peau de médicaments, en particulier pour de la médecine sportive, mais est également envisagée maintenant pour une meilleure pénétration d'un cosmétique. Dans un mode de réalisation, ce dispositif de traitement ionophorèse transcutanée comprend deux électrodes à potentiel électrique différent qui peuvent être agencées sur les embouts de massage et/ou sur la surface d'application de la tête de massage. Les réglages de l'appareil de massage selon l'invention, en particulier lorsque celui-ci comprend également un système de distribution de produits cosmétique, permettront de réaliser le traitement par ionophorèse transcutanée avant et /ou pendant l'application du du produit cosmétique.

La description suivante met en évidence les caractéristiques et avantages de l'appareil de massage objet de l'invention, laquelle s'appuie sur des figures parmi lesquelles :
- La figure 1 est une vue en perspective de l'appareil de massage selon un mode de réalisation ;
- Les figures 2 à 5 mettent en évidence les caractéristiques du mécanisme de transmission de l'appareil de massage selon ce mode de réalisation illustré en figure 1 ;
- Les figures 6 à 8 mettent en évidence les caractéristiques d'une variante de conception du mécanisme de transmission de l'appareil de massage ;
- Les figures 9 et 10 schématisent les trajectoires des embouts sur les têtes de massage grâce à la conception du mécanisme de transmission de l'appareil de massage selon l'invention ;
- Les figures 11A, 11B et 11C schématisent des variantes de formes d'embouts de massage ;
- Les figures 12 à 27 schématisent des variantes de système de guidage, de système de retour des embouts de massage et/ou de système de rapprochement des embouts de massage envisageables pour diverses variantes de conception du mécanisme de transmission ;
- La figure 28 schématise un système vibratoire sur l'appareil de massage ;
- La figure 29 schématise une peau souple agencée sur une tête de massage ;
- La figure 30 schématise un système d'émission d'ondes sur une tête de massage ;
- La figure 31 schématise une couronne agencée sur une tête de massage ;
- Les figures 32 et 33 schématisent un mécanisme de transmission pour l'entraînement de la couronne de la figure 31 ;
- Les figures 34 à 37 schématisent un système de distribution de produit cosmétique sur l'appareil de massage ;
- La figure 38 schématise un système d'assemblage amovible entre la tête de massage et le corps de l'appareil de massage et ;
- La figure 39 schématise un dispositif de traitement ionophorère transcutanée sur l'appareil de massage ;
- Les figures 40 à 43 schématisent quatre variantes de mécanisme de transmission sur la tête de massage ;
- Les figures 44A - 44D schématise le rapprochement et le retour d'un embout de massage avec les variantes de mécanisme de transmission des figures 40 à 43.

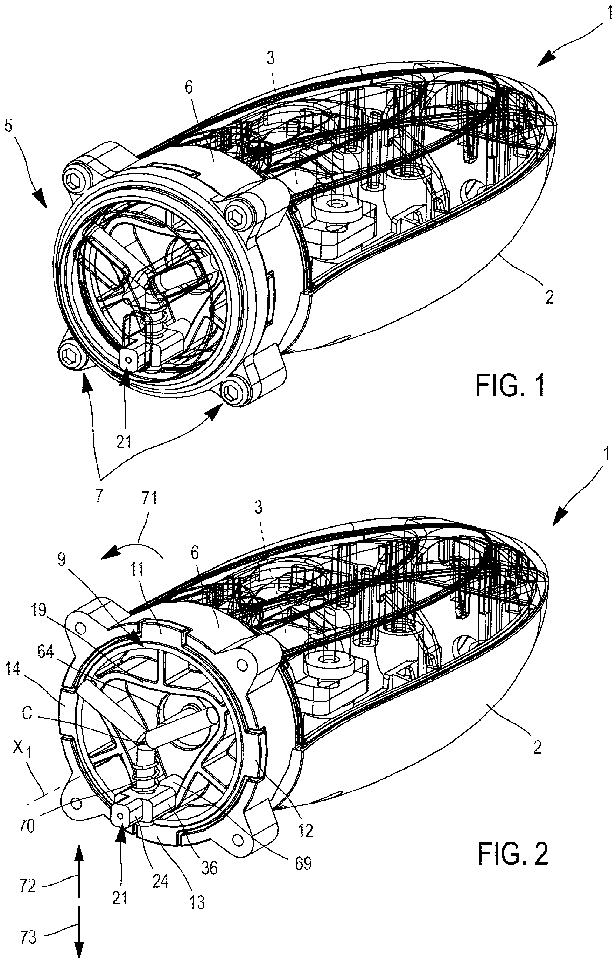

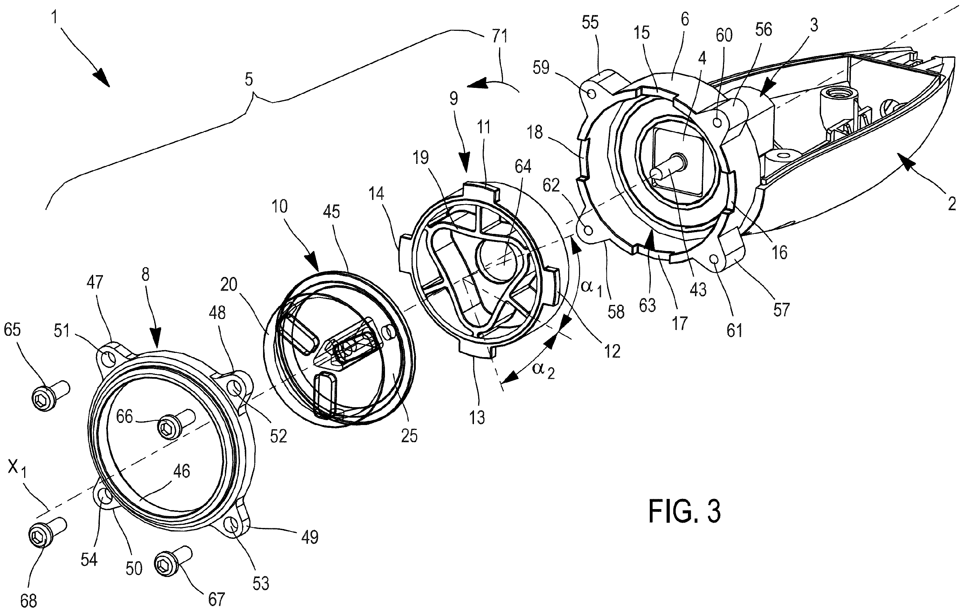

Tel qu'illustré sur la figure 1 à 3, l'appareil de massage 1 comprend un corps 2 à l'intérieur duquel est agencé un moteur 3 alimenté électriquement par raccordement soit à une source électrique externe, au moyen d'une prise électrique et d'un transformateur basse tension, soit à source électrique interne du type batterie rechargeable ou piles jetables. L'appareil de massage 1 comprend également un motoréducteur 4, illustré en figures 3 à 5, agencé en sortie du moteur 3 pour réduire sa vitesse de rotation et l'adapter au besoin d'utilisation.

Tel qu'illustré sur les figures 1 à 5, l'appareil de massage 1 comprend également une tête de massage 5. Le corps 2 comprend à son extrémité un carter 6 permettant la réception des éléments de la tête de massage 5 qui est maintenue assemblée avec ledit carter 6 grâce à des moyens d'assemblage 7. Cette tête de massage 6 comprend un capot 8, une pièce de transmission 9 et une pièce de guidage 10. La pièce de transmission 9 comprend quatre oreilles 11, 12, 13, 14 configurées pour être engagées dans quatre encoches 15, 16, 17, 18 respectives agencées sur le carter 6, ce qui permet le blocage en rotation de la pièce de transmission 9 sur le carter 6, selon un axe X₁. Cette pièce de transmission 9 comprend une came 19 qui présente une forme triangulaire. La pièce de guidage 10 est de forme cylindrique et présente une surface d'application 20 perpendiculaire à l'axe X₁. Cette surface d'application 20 est positionnée parallèlement à la surface de la peau lors de l'utilisation de l'appareil de massage 1.

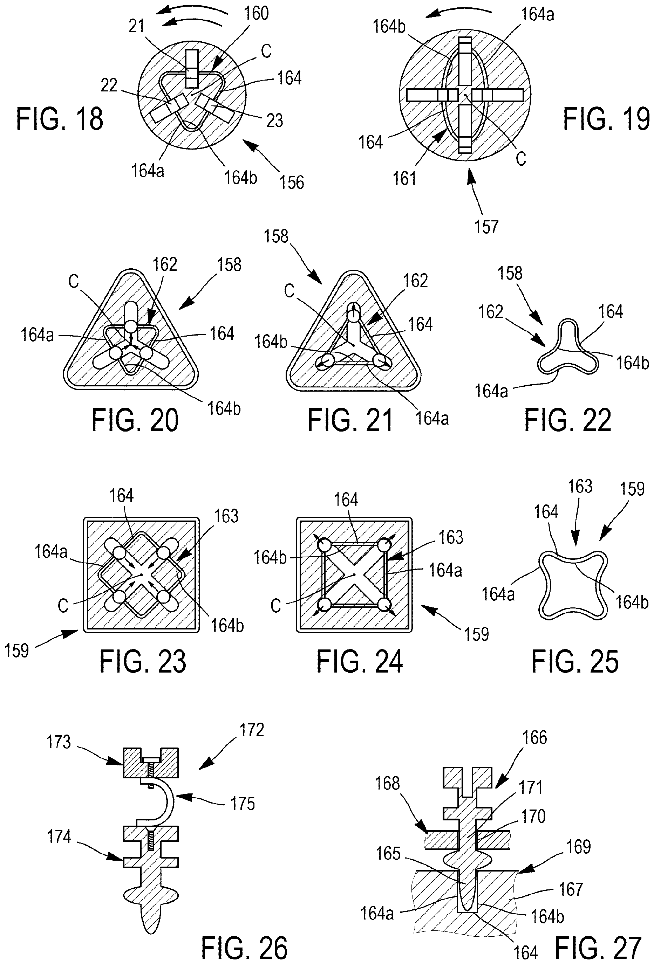

Sur ces figures 1 à 5, est illustré un seul embout de massage 21. On comprend toutefois en regard de ces figures 1 à 5, et comme cela est schématisé par exemple sur la figure 18 dans une variante, que la tête de massage 5 selon ce mode de réalisation comprend trois embouts de massage 21, 22, 23 uniformément répartis et identiques. L'embout de massage 21 comprend une portion externe 24 qui s'étend perpendiculairement à la surface d'application 20, vers l'extérieur de celle-ci. Cette portion externe 24 vient en contact avec la peau de l'utilisateur lors de l'application de l'appareil de massage 1.

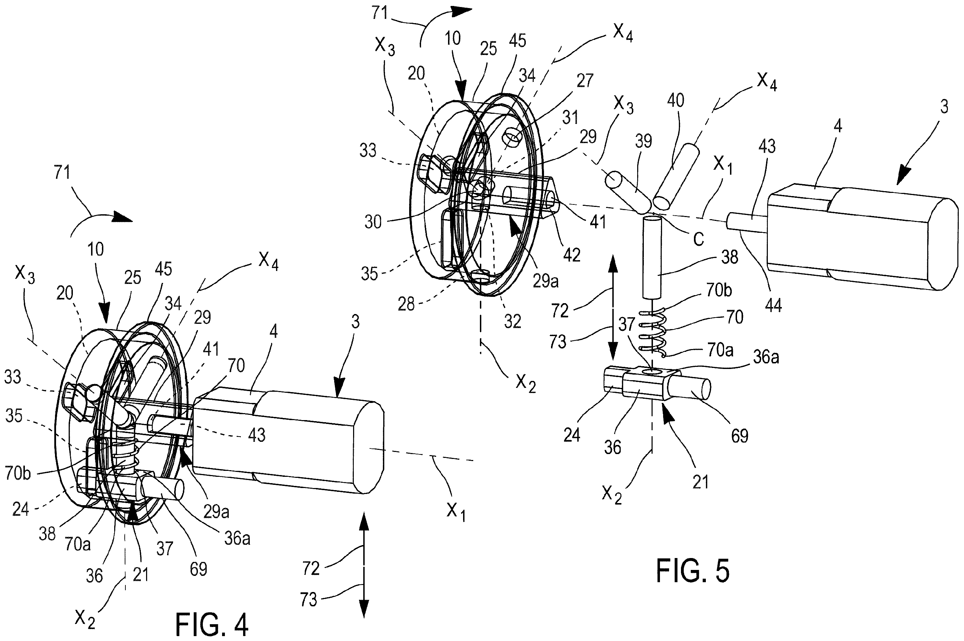

Tel qu'illustré sur les figures 1 à 5, la pièce de guidage 10 comprend une portion cylindrique 25 sur laquelle sont agencés trois trous débouchant 26, 27, 28. Cette pièce de guidage 1 comprend également une portion axiale 29 qui comprend trois trous 30, 31, 32 agencés respectivement dans les mêmes axes X₂, X₃, X₄ que les trous débouchant 26, 27, 28. Cette portion axiale 29 comprend une forme triangulaire dont les faces 29a, 29b, 29c comprennent respectivement les trois trous 30, 31, 32. Sur la surface d'application 20 sont agencées trois lumières 33, 34, 35 qui permettent le guidage des portions externes 24 des embouts 21, 22, 23. L'embout de massage 21 comprend également une portion centrale 36 qui comprend un orifice débouchant 37 agencé selon l'axe X₂. La tête d'assemblage 5 comprend trois goupilles 38, 39, 40 agencées respectivement selon les axes X₂, X₃, X₄ et montées serrées dans les trous 26, 27, 28, 30, 31, 32 au travers desquels elles pénètrent. On constate sur les figures 4 et 5 que la goupille 38 passe également au travers du trou 37 sur la portion centrale 36 de l'embout de massage 21. Ainsi l'embout de massage 21 est monté en translation selon l'axe X₂ sur la pièce de guidage 10. Il en est de même pour les embouts 22, 23 selon les axes X₃ et X₄.

Tel qu'illustré sur les figures 3 à 5, la portion axiale 29 de la pièce de guidage 10 comprend au niveau de son extrémité interne un trou 41 agencé selon l'axe X₁ et comprenant un méplat 42. Le moteur 3 comprend en sortie du motoréducteur 4, un arbre 43 agencé selon l'axe X₁ et comprenant également un méplat 44. L'engagement de l'arbre 43 dans le trou 41 de la portion axiale 29 permet l'entraînement en rotation selon l'axe X₁ de la pièce de guidage 10 sous l'action du moteur 3.

Tel qu'illustré sur les figures 1 à 5, la pièce de guidage comprend une portion épaulée 45. Le capot 8 comprend une ouverture circulaire 46 configurée pour recevoir la portion cylindrique 25 de la pièce de guidage 10 et venir en butée contre la portion épaulée 45. Le capot 8 comprend quatre oreilles 47, 48, 49, 50 munies chacune d'un trou débouchant 51, 52, 53, 54. Le carter 6 comprend également quatre oreilles 55, 56, 57, 58 munies chacune d'un taraudage 59, 60, 61, 62. L'assemblage est réalisé en engageant la pièce de transmission 9 dans la chambre 63 du carter 6, l'arbre 43 du moteur 3 passant au travers d'un orifice 64 agencé sur la pièce de transmission 9. Puis en engageant la pièce de guidage 10, sur laquelle sont montés les embouts de massage 21, 22, 23, avec l'arbre 43 du moteur 3. Enfin, le capot 8 est plaqué sur le carter 6 et maintenu assemblé au moyen de vis de fixation 65, 66, 67, 68 passant au travers des orifices 51, 52, 53, 54 sur le capot 8 et vissées dans les taraudages 59, 60, 61, 62 du carter 6. Un assemblage par vissage permet de remplacer facilement la tête de massage 5 par une autre tête de massage identique ou différente, comme précisé ci-après.

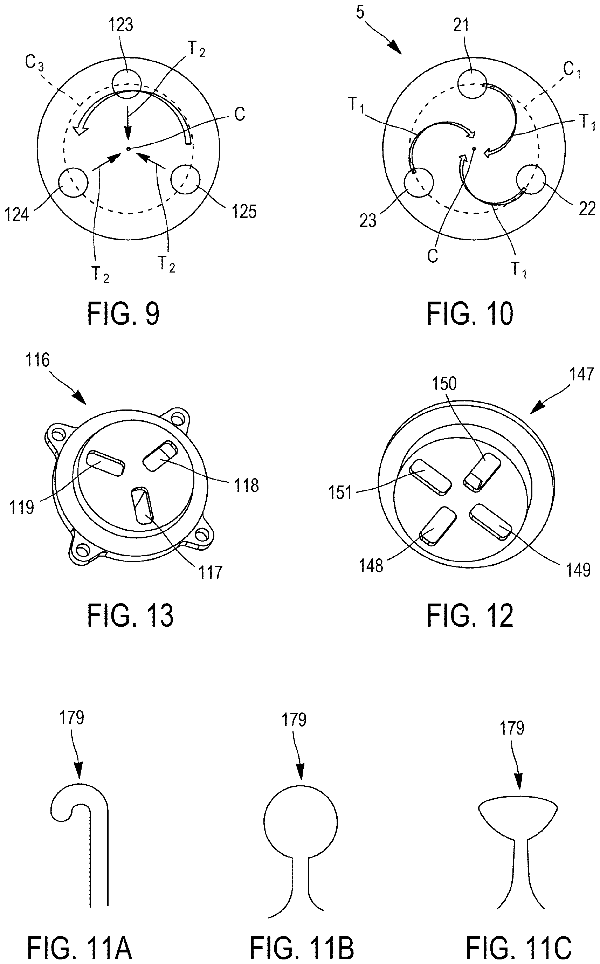

Tel qu'illustré en figures 2 à 5, l'embout de massage 21 comprend une portion interne 69. Lorsque la tête de massage 5 est assemblée sur le corps 2, cette portion interne 69 est positionnée à l'intérieur de la came 19 de la pièce de transmission 9. Cette portion interne 69 est maintenu en appui contre la came au moyen d'un ressort 70 monté sur la goupille 38 et appuyant à sa première extrémité 70a, sur la face interne 36a de la portion centrale 36 de l'embout de massage 21 et, à sa seconde extrémité 70b, sur la face 29a correspondant de la portion axiale 29 de la pièce de guidage 10. Lorsque la pièce de guidage 10 est entraînée en rotation selon l'axe X₁ par le moteur 3 dans le sens de la flèche 71, l'embout de massage 21 tourne avec celle-ci. La came 19 exerce un effort contre la portion interne 69 et pousse l'embout de massage 21 dans le sens de la flèche 72 sur une première portion angulaire α₁, ce qui permet de translater l'embout de massage 21 selon l'axe X₂ dans le sens de la flèche 72, vers le centre C défini par l'intersection de l'axe X₁ avec les axes X₂, X₃, X₄. Puis, sur une seconde portion angulaire α₂, la came 19 cesse d'exercer cet effort sur la portion interne 69. Le ressort 70 qui exerce une fonction de rappel ou de retour, pousse contre la portion centrale 36 de l'embout de massage 21 dans le sens de la flèche 73 et le translate selon l'axe X₂ dans le sens de cette flèche 73, pour l'éloigner du centre C. La rotation de la pièce de guidage 10 autour de l'axe X₁ combinée à la translation de l'embout de massage 21 selon l'axe X₂ permet donc à cet embout de massage 21 de se rapprocher puis de s'éloigner du centre C en effectuant un mouvement de translation circulaire ou curviligne. On comprend que les deux autres embouts de massage 22, 23 subissent les mêmes mouvements en synchronisation avec l'embout de massage 21. Ainsi les trois embouts de massage 21, 22, 23 sont disposés sur un même cercle virtuel C₁ de centre C et se déplacent en effectuant une translation curviligne T1 passant par le centre C, tel que schématisé en figure 10. Cela permet de réaliser un pincé tournant de la peau au moyen de la tête de massage 5.

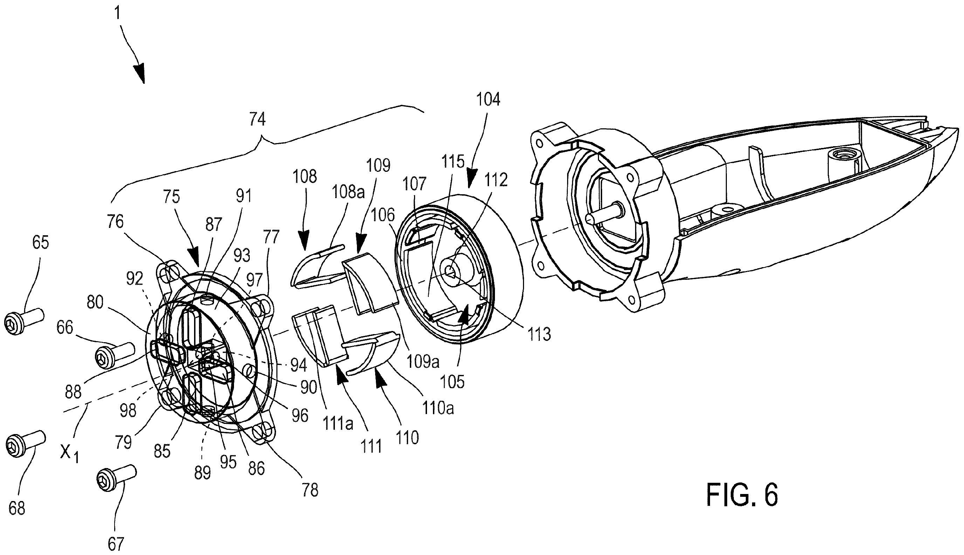

Dans une variante de réalisation, illustrée par les figures 6 à 8, seule la tête de massage 5 comprend une conception différente, le corps 2 de l'appareil de massage 1 présentant une conception identique à celle illustrée en figures 1 à 3. On pourrait toutefois envisager également des variantes de conception du corps 2, sans sortir du cadre de l'invention telle que définie dans les revendications.

Selon ces figures 6 à 8, la tête de massage 74 comprend une pièce de guidage 75 qui constitue également un capot. Cette pièce de guidage 75 comprend quatre oreilles 76, 77, 78, 79 qui permettent d'assembler ladite pièce de guidage 75 sur le carter 6 du corps 2, de manière similaire au capot 8 de mode réalisation illustré en figures 1 à 5, au moyen des vis de fixation 65, 66, 67, 68. Ainsi la pièce de guidage 75 est immobilisée sur le corps 2 de l'appareil de massage 1. Cette pièce de guidage comprend une surface d'application 80 qui est position parallèlement à proximité de la surface de la peau lors de l'application de l'appareil de massage 1.

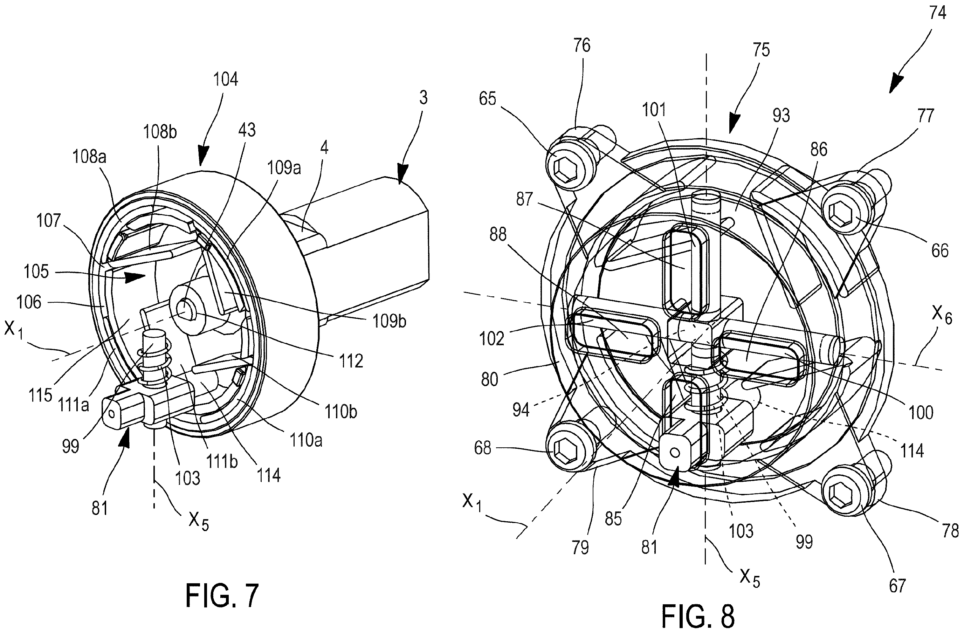

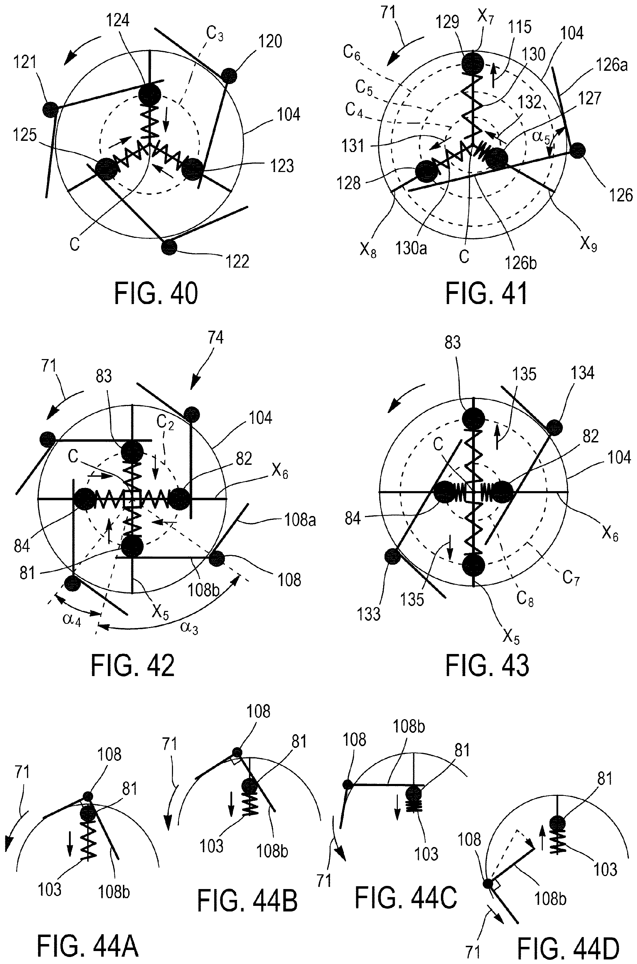

En regard des figures 6 à 8, on comprend qu'à la différence de la tête d'assemblage 6 des figures 1 à 5, la tête de massage 74 dispose de quatre embouts de massage, un seul embout de massage 81 étant représenté pour faciliter la compréhension des figures 6 à 8. Ces quatre embouts de massage 81, 82, 83, 84 sont toutefois schématisés en figures 42 et 43. Ces embouts de massage 81, 82, 83, 84 sont répartis uniformément sur la pièce de guidage 75 et montés en translation sur cette pièce de guidage 75 selon deux axes X₅, X₆ perpendiculaires entre eux et disposés dans un même plan perpendiculaire à l'axe X₁ de rotation de l'arbre 43 du moteur 3. Le montage en translation des embouts de massage 81, 82, 83, 84 sur la pièce de guidage 75 est réalisé de manière identique à celui des embouts de massage 21, 22, 23 sur la pièce de guidage 10, à la différence que les éléments sont au nombre de quatre au lieu de trois. Ainsi, la pièce de guidage 75 comprend quatre lumières 85, 86, 87, 88 uniformément réparties sur sa face d'application 80, quatre trous débouchant 89, 90, 91, 92 sur sa portion cylindrique 93 et, sa portion axiale 94 présente une forme carrée à quatre face munies chacune d'un trou 95, 96, 97, 98. La tête de massage 74 comprend en outre quatre goupilles 99, 100, 101, 102 montées serrées au travers des trous 89, 90, 91, 92, 95, 96, 97, 98 et recevant chacune un ressort 103, un seul étant représenté sur les figures 7 et 8.

Tel qu'illustré en figures 6 à 8, la tête de massage 74 comprend une pièce de transmission 104 de forme cylindrique et présentant une chambre 105. Cette pièce de transmission 104 comprend une rainure circulaire 106 sur sa face interne qui comprend des encoches 107. La tête de massage 74 comprend également quatre cames 108, 109, 110, 111. Les premières parties 108a, 109a, 110a, 111 a s'engagent dans la rainure circulaire 106 et dans les encoches 107, ce qui assure l'immobilisation de ces cames sur la pièce de transmission 104. Les secondes parties 108b, 109b, 110b, 111 b s'étendent à l'intérieur de la chambre 105 de la pièce de transmission 104 en formant un carré ou un losange comme on le constate sur les figures 6, 7 et 42.

Tel qu'illustré en figures 6 et 8, la pièce de transmission 104 comprend un orifice débouchant 112 d'axe X1 qui présente un méplat 113 permettant la réception de l'arbre 43 du moteur lorsque la pièce de transmission 104 est engagée dans la chambre 63 du carter 6 sur le corps 2. La rotation de l'arbre 43 entraîne celle de la pièce de transmission 104 et des cames 108, 109, 110, 111 selon l'axe X₁.

Lorsque la pièce de guidage 75 est assemblée sur le carter 6, la portion interne 114 de l'embout de massage 81 est positionnée dans la chambre 105 de la pièce de transmission 104 et reste en appui soit contre la face interne cylindrique 115 de cette chambre 105 soit contre les secondes parties 108b, 109b, 110b, 111 b des cames 108, 109, 110, 111 lors de leur rotation selon l'axe X₁. Il en est de même pour les autres embouts 82, 83, 84 schématisés en figure 42. Comme le schématisent les figures 42 et 44A - 44D, lors de la rotation de la pièce de transmission 104 dans le sens de la flèche 71 sur une portion angulaire α₃, la seconde partie 108b de la came 108 exerce un effort contre l'embout de massage 81 dans le sens de la flèche 72 et comprime le ressort 99, ce qui permet la translation de l'embout de massage 81 selon l'axe X₅, vers le centre C. Lorsque une portion angulaire α₄ suivante, la came 108 quitte le contact avec l'embout de massage 81, ce qui permet au ressort 103 de pousser contre l'embout de massage 81 dans le sens de la flèche 73 et de translater cet embout de massage 81 qui s'éloigne du centre C selon l'axe X₅ jusqu'à revenir en appui contre la face interne cylindrique 115. Il en est de même pour les autres embouts de massage 82, 83, 84 qui se rapprochent et s'éloignent du centre C selon les axes X₅, X₆, de manière synchronisée et identique à l'embout de massage 81. Ainsi, ces embouts de massage 81, 82, 83, 84 sont disposés sur un même cercle virtuel C₂, schématisé en figure 42, et se déplacent donc de manière synchronisée en translation radiale de centre C, ce qui permet de réaliser un pincé simple au moyen de cette tête de massage 74.

De multiples variantes de conception sont envisageables pour la mise en oeuvre d'un mécanisme de transmission sur la tête de massage permettant aux embouts de massage de réaliser une translation radiale ou une translation circulaire dirigée vers le centre C.

Sur la figure 13, est illustrée une pièce de guidage 116 de conception similaire à la pièce de guidage 75 des figures 6 à 8, mais ne présentant que trois lumières 117, 118, 119 pour le guidage de trois embouts de massage. Dans ce cas, la pièce de transmission 104 ne reçoit que trois cames 120, 121, 122, schématisées en figure 40, similaires aux cames 108, 109, 110, 111 et disposées avec une répartition uniforme. Comme le schématise les figures 9 et 40, les embouts de massage 123, 124, 125 sont disposés selon un cercle virtuel C₃ et se déplacent en translation radiale T₂ de centre C.

Sur la figure 41 est schématisée une seule came 126 qui est montée sur la pièce de transmission 104 et trois embouts de massage 127, 128, 129 montés sur une pièce de guidage similaire à la pièce de guidage 116 de la figure 13. Cette came 126 est similaire aux cames 108, 109, 110, 111 des figures 6 à 8, mais présente une seconde partie 126b de longueur plus importante et formant un angle α₅ avec la première partie 126a dimensionné en sorte que durant la rotation de la pièce de transmission 104 selon la flèche 71, la came 126 soit en contact simultanément avec deux embouts de massage 127, 128, mais avec un décalage. Ainsi, tel qu'illustré sur la figure 41, le premier embout de massage 129 est dégagé de la came 126 et poussé sur l'axe X₇ par le premier ressort 130 contre la face interne cylindrique 115 de la pièce de transmission 104. Le second embout de massage 128 est en appui contre la came 126 et translate sur l'axe X₈ dans le sens de la flèche 131 sous l'effet d'un ressort 130a. Et le troisième embout de massage 127 est en appui contre la came 126 et translate dans le sens de la flèche 132 selon l'axe X₉. Ainsi de suite durant la rotation de la pièce de transmission 104 et de la came 126. Selon cette conception, les embouts de massage 127, 128, 129 sont disposés selon des cercles virtuels C₄, C₅, C₆ de centre C et translate radialement vers le centre C, avec un décalage entre ces embouts de massage 127, 128, 129. On réalise ainsi un pincé particulier au moyen d'une telle tête de massage.

Sur la figure 43 sont illustrées deux cames 133, 134 montées sur la pièce de transmission 104 et disposées en vis-à-vis. La pièce de guidage est identique à la pièce de guidage 75 des figures 6 à 8 et comprend les quatre embouts de massage 81, 82, 83, 84. Ces cames 133, 134 activent simultanément deux 82, 84 des quatre embouts de massage disposés en vis-à-vis puis, alternativement, les deux autres embouts de massage 81, 83 en vis-à-vis, durant la rotation de la pièce de transmission 104. Ainsi, les deux embouts de massage 81, 83 en vis-à-vis sont sur un même cercle virtuel C₇ de centre C et translatent radialement selon l'axe X₅ dans le sens de la flèche 135 pendant que les deux autres embouts de massage 82, 84 en vis-à-vis, disposés sur un même cercle C₈ de centre C, translatent radialement selon l'axe X₆ dans le sens inverse. Ainsi de suite alternativement. On réalise ainsi un pincé alternatif au moyen d'une telle tête de massage.

Les cames 108, 109, 110, 111 illustrées en figures 6 et 8 sont par exemple mises en oeuvre au moyen de lamelles métalliques ou en plastique dont les caractéristiques dimensionnelles sont configurées pour disposer de souplesse entre la première partie 108a, 109a, 110a, 11a et la seconde partie 108b, 109b, 110b, 111b. Ainsi ces cames 108, 109, 110, 111 permettent d'amortir les embouts de massage 81, 82, 83, 84 lorsque les efforts exercés par ces embouts de massage sur la peau, durant le pincement, sont trop importants. Cela évite donc que le massage soit douloureux. Il en est de même pour toutes les variantes précitées utilisant un tel type de came composé de lamelles.

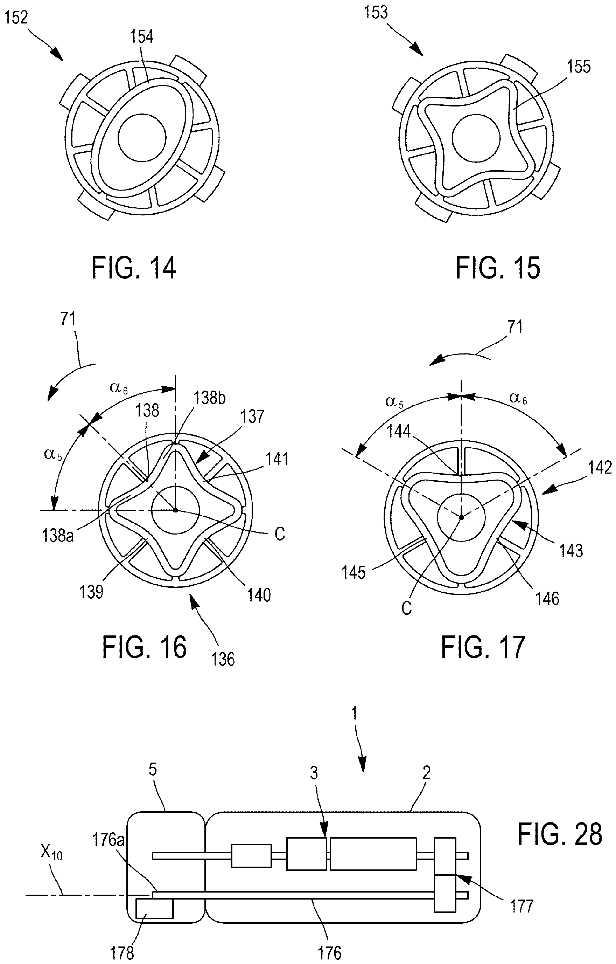

On peut toutefois envisager de remplacer les cames 108, 109, 110, 111 et la pièce de transmission 104 sur le mode illustré en figure 6 à 8, par une pièce de transmission 136, illustrée en figure 16, qui comprend une came 137 présentant une forme étoilée, voire un carré ou un losange, avec quatre face 138, 139, 140, 141. Lors de la rotation de la pièce de transmission 136 dans le sens de la flèche 71 sur une première portion angulaire α₅, la première partie 138a de la face 138 permet de rapprocher l'embout de massage vers le centre C, tandis que sur une portion angulaire suivante α₆, la seconde partie 138b de la face 138 autorise l'éloignement de l'embout de massage du centre C, sous l'action du ressort. Il en est de même avec les autres faces 139, 140, 141. Le mouvement des embouts de massage 81, 82, 83, 84 sont donc les mêmes que sur la figure 42.

Il en est de même avec une variante de conception à trois embouts de massage. Ainsi, sur la figure 17, la pièce de transmission 142 présente une came 143 de forme triangulaire avec trois faces 144, 145, 146 remplissant les mêmes fonctions que précédemment.

Sur la figure 12, la pièce de guidage 147 fonctionne de manière similaire à la pièce de guidage 10 des figures 1 à 5, à la différence que cette pièce de guidage 147 comprend quatre lumières 148, 149, 150, 151 permettant la réception de quatre embouts de massage. Dans ce cas, on utilise par exemple les pièces de transmission 152, 153 illustrées respectivement en figures 14 et 15 et de conception semblable à la pièce de transmission 9 des figures 1 à 5, à la différence qu'elles agissent sur quatre embouts de massage. La pièce de transmission 152 de la figure 14 comprend une came 154 présentant une forme elliptique qui permet d'obtenir en combinaison avec la pièce de guidage 147, une trajectoire des embouts de massage selon une translation circulaire de centre C, avec une alternance entre les embouts disposés en vis-à-vis. La pièce de transmission 153 de la figure 15 comprend une came 155 en forme étoilée, voire carrée ou en losange, qui permet d'obtenir en combinaison avec la pièce de guidage 147, une trajectoire des embouts de massage selon une translation circulaire de centre C, de manière comparable à celle illustrée en figure 10 pour les trois embouts de massage 21, 22, 23.

Sur les figures 18 à 25 sont schématisées diverses conceptions de mécanismes de transmission 156, 157, 158, 159 sur une tête de massage dont le principe de fonctionnement est similaire aux diverses variantes déjà décrites, mais pour lesquelles les ressorts 70, 103 illustrés en figures 4, 5, 6 et 7, précédemment utilisés pour pousser contre les embouts de massage et les éloigner du centre C, sont supprimés. Selon ces variantes de conception illustrées en figure 18 à 25, la fonction de retour des embouts de massage est directement assurée par la came 160, 161, 162, 163 de la pièce de transmission qui comprend une rainure 164. Comme illustré en figure 27, la partie inférieure 165 de l'embout de massage 166 est positionné dans la rainure 164 de la came 167. Durant la rotation de la pièce de guidage 168 relativement à la pièce de transmission 169, sur une première portion angulaire, la face externe 164a de cette rainure 164 pousse contre l'embout de massage 166 pour le translater vers le centre C, radialement ou circulairement selon la conception de la tête de massage. Au contraire, sur une seconde portion angulaire, la face interne 164b de cette rainure 164 pousse contre l'embout de massage 166 pour l'éloigner en translation du centre C, radialement ou circulairement selon la conception de la tête de massage. Comme on le constate sur les figures 18 à 25, cette rainure 164 peut avoir une forme de triangle, d'ellipse, de losange, d'étoile à trois ou quatre branches.

Comme on le constate en figure 27, le guidage en translation de l'embout 166 est assuré, d'une part, par une lumière 170 agencée sur la pièce de guidage 168 qui reçoit une partie centrale 171 de l'embout de massage 166 et, d'autre part, par la partie inférieure 165 de l'embout de massage 166 guidée dans la rainure 164 de la came 167. Ainsi, cette variante permet de supprimer les goupilles de guidage décrites pour les variantes des figures 1 à 5 et 6 à 8.

Dans une variante de réalisation illustrée en figure 26, l'embout de massage 172 est composé de d'une partie supérieure 173 et d'une partie inférieure 174 connectées entre elles par une partie centrale 175 qui est sensiblement souple et joue la fonction d'amortisseur permettant de limiter les efforts sur la peau lors d'un pincement. D'autres variantes sont envisageables selon ce principe de fonctionnement, sans sortir du cadre de l'invention telle que définie dans les revendications.

D'autres caractéristiques sont envisageables sur l'appareil de massage 1 objet de l'invention telle que définie dans les revendications. Tel qu'illustré en figure 28, l'appareil de massage 1 comprend un arbre 176 qui est entraîné en rotation selon un axe X₁₀ par le moteur 3, au moyen d'un système d'engrenage 177 connu de l'homme du métier. L'extrémité 176a de de cet arbre 176 est agencée dans la tête de massage 5 et reçoit une masselotte 178 qui tourne en balourd autour de l'axe X₁₀ et permet de faire vibrer la tête de massage 5 durant l'application de l'appareil de massage 1. D'autres variantes de système vibratoire restent envisageables. On peut notamment envisager de faire vibrer uniquement les embouts de massage.

Tel qu'illustré sur les figures 11A - 11C, la partie externe 179 des embouts de massage peut présenter des formes variables comme, par exemple, une forme de crochet en figure 11A, une forme de boule en figure 11B et une forme plus ou moins aplatie en figure 11C. D'autres formes restent envisageables. En outre, cette partie externe 179, voire l'embout de massage complètement, est dans une matière telle que de l'élastomère permettant, en combinaison avec la forme, de mieux accrocher la peau lors de l'application de l'appareil de massage 1.

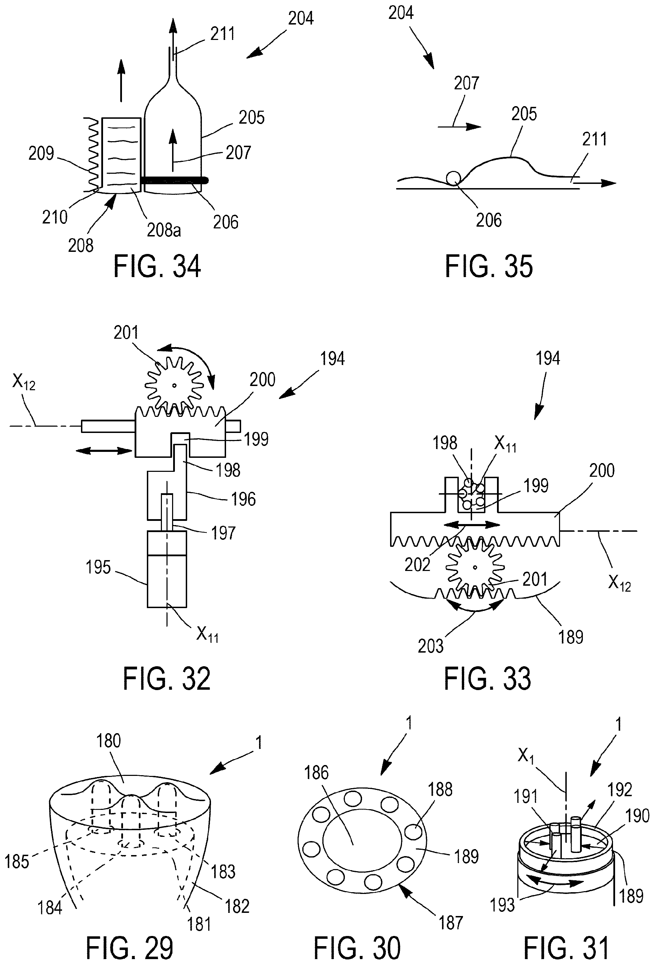

Dans une variante illustrée sur la figure 29, l'appareil de massage 1 comprend une peau souple 180 qui est agencée au-dessus de la surface d'application 181 de la tête de massage 182 et recouvre les embouts de massage 183, 184, 185. Cette peau souple 180 évite notamment que les embouts ne paraissent trop agressifs et améliore l'adhérence sur la peau grâce à un contact surfacique.

Dans une variante illustrée en figure 30, l'appareil de massage 1 comprend au niveau de la surface d'application 186 sur la tête de massage 187, des diodes luminescentes 188 qui sont commandées par un boîtier électronique (non illustré) agencé à l'intérieur du corps 2. Ces diodes luminescentes 188 pourront être allumées soit automatiquement lors de l'actionnement de la tête de massage 188, soit séparément au moyen du bouton de commande distinct (non illustré). Les diodes luminescentes 188 seront disposées par exemple sur le bord périphérique 189 de la surface d'application 186, voire réparties sur ladite surface d'application 186 en dehors des trajectoires des embouts de massage. On pourra utiliser des diodes luminescentes de différentes couleur selon la longueur d'onde souhaitée et/ou le traitement recherché, voire prévoir un boîtier de commande permettant de modifier la longueur d'onde de ces diodes luminescentes 188.

Dans une variante illustrée sur la figure 31, l'appareil de massage 1 comprend une couronne 189 qui est agencée en rotation selon l'axe X₁ et s'étend par rapport à la surface d'application 190 de sorte que les extrémités 191 des embouts de massage soient positionnées dans le même plan que le bord périphérique 192 de la couronne 189. Cette couronne 189 est de préférence actionnée en rotation selon un mouvement alternatif d'axe X₁ dans les sens de la flèche 193. Pour cela, l'appareil de massage 1 comprend un mécanisme de transmission 194 illustré en figure 32 et 33. Ce mécanisme de transmission 194 comprend un motoréducteur 195 qui est disposé dans le corps 2 de l'appareil de massage 1. Ce motoréducteur 195 peut être différent ou le même que le motoréducteur 4 entrainé par le moteur 3. Il peut en outre être entraîné par le même moteur 3 par le biais d'un système d'engrenage (non illustré) voire par un moteur indépendant disposé dans le corps 2. Un excentrique 196 est agencé sur l'arbre 197 du motoréducteur 195. Cet excentrique comprend un doigt 198 décalé par rapport à l'axe X₁₁ de rotation de l'arbre 197. Ce doigt 198 est positionné dans une rainure 199 agencée sur une crémaillère 200 montée en translation selon un axe X₁₂ et engrenant avec un pignon 201 qui engrène lui-même avec la couronne 189, comme illustré en figures 32 et 33. Ainsi le doigt 198 dispose d'un certain degré de liberté dans la rainure 199 lors de sa rotation autour de l'axe X₈, ce qui permet de transmettre un mouvement alternatif selon la flèche 202 à la crémaillère et donc, un mouvement de rotation alternatif selon la flèche 203 au pignon 201 et à la couronne 189. L'avantage de ce mécanisme transmission 194 est de pouvoir modifier facilement l'angle d'inclinaison entre le doigt 198 et la rainure 199, ce qui permet d'assembler la tête de massage 5 sur le corps 2 avec différentes orientations angulaires, par exemple dans l'axe du corps 2 ou avec une inclinaison à 45 degrés par rapport au corps 2.

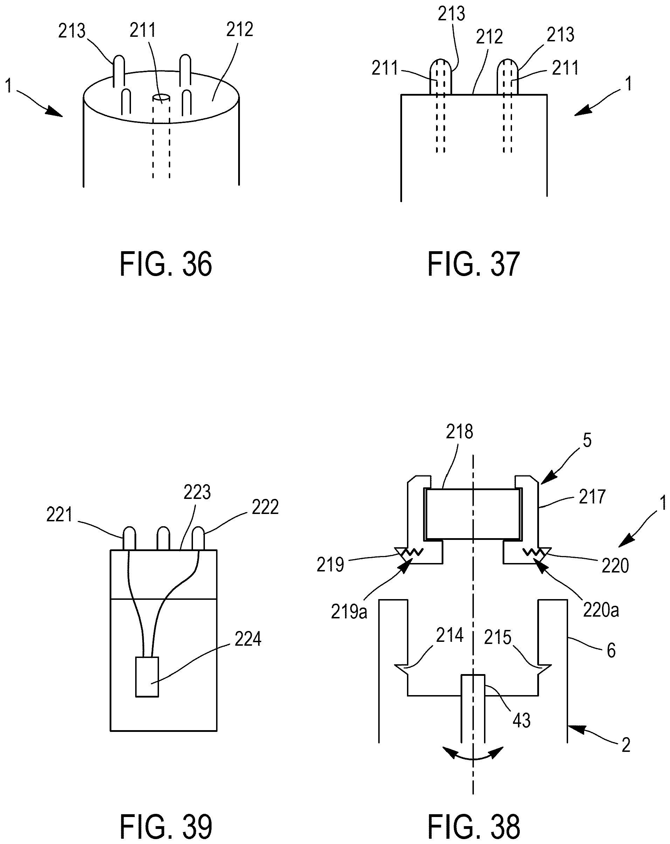

Dans une variante illustrée en figures 34 et 35, on prévoit sur l'appareil de massage 1 un système de distribution de produit cosmétique 204 qui comprend un réservoir de produit 205 souple sur lequel appuie une barrette 206 qui se déplace en translation selon la flèche 207 le long du réservoir de produit 205. Un système d'actionnement 208 permet de déplacer la barrette 206 selon la flèche 207. Ce système d'actionnement 208 est par exemple constitué d'un bouton 208a solidaire de la barrette 206 et translatant dans le sens de cette flèche lors d'une action manuelle. Une pièce crantée 209 permet de maintenir un cran 210 sur le bouton 208a lors de chaque impulsion sur celui-ci pour maintenir en position la barrette 206 au fur et à mesure de son avancement sur le réservoir de produit 205. Le système de distribution 204 comprend un tuyau 211 dont l'agencement permet de distribuer le produit cosmétique au niveau de la surface d'application 212, soit entre les embouts 213 soit au travers des embouts 213, comme illustré en figures 36 et 37. D'autres variantes de système de distribution de produit cosmétique sont envisageables. On peut notamment prévoir un système d'actionnement automatique permettant de distribuer de manière continue et régulière, par exemple au moyen d'une pompe, le produit cosmétique. On peut également prévoir un système de distribution de produit cosmétique de manière naturelle, le produit cosmétique étant diffusé régulièrement grâce à des phénomènes physiques ne nécessitant pas un actionnement extérieur.

Tel qu'illustré au regard de variantes de réalisation de l'appareil de massage 1 selon les figures 1 à 5 et 6 à 8, la tête de massage 5 peut être montée et démontée rapidement en retirant les vis de fixation 65, 66, 67, 68. Cela permet de changer facilement de tête d'assemblage 4. Pour améliorer la rapidité d'assemblage de la tête de massage 5 et son caractère amovible, on peut envisager un système de fixation amovible plus performant entre la tête de massage 5 et le corps 2. Par exemple, comme illustré sur la figure 38, le carter 6 du corps 2 peut comprendre des encoches 214, 215 et la tête de massage 216 comprend une pièce 217 qui reçoit le mécanisme de transmission 218 du mouvement des embouts de massage connecté à l'arbre 43 du moteur 3, cette pièce 217 comprenant des crans 219, 220, des moyens de rétractation 219a, 220a des crans 219, 220 permettant de les escamoter pour leur positionnement dans les encoches 214, 215 et aussi leur retrait de ces encoches 214, 215.

Dans une variante de réalisation illustrée en figure 39, l'appareil de massage 1 comprend deux électrodes 221, 222 qui présentent un potentiel électrique différent. Ces électrodes 221, 222 peuvent consister en deux des embouts de massage ou être agencées sur la surface d'application 223. Ces électrodes 221, 222 sont alimentées par une source électrique 224 agencé dans le corps 2. Cette conception permet de réaliser un traitement par ionophorèse transcutanée avant ou pendant l'application d'un produit cosmétique sur la peau, ce qui permet d'accélérer la pénétration du produit cosmétique. Ce produit cosmétique peut être distribué de manière naturelle, manuelle ou automatique par l'appareil de massage 1, voire appliqué directement sur la peau par l'utilisateur.

Ces caractéristiques complémentaires précitées des différentes variantes de réalisation de l'appareil de massage 1 pourront éventuellement être mise en oeuvre en combinaison avec l'un ou l'autre des mécanismes de transmission sur la tête d'assemblage tels que décrits ci-dessus, voire avec d'autres variantes envisageables dans le cadre de l'invention telle que définie dans les revendications. A titre d'exemple, on peut envisage de concevoir une couronne 189 et une peau souple 180 au moyen d'une même pièce.

## Revendications

1. Appareil de massage (1) comprenant un corps (2) qui comporte des moyens de motorisation (3, 4) et une tête de massage (5), des moyens d'assemblage étant agencés entre le corps et la tête de massage, ladite tête de massage comportant une surface d'application et au moins trois embouts de massage qui s'étendent vers l'extérieur de la surface d'application,
où
les embouts de massage sont définis sur la surface d'application selon des cercles virtuels concentriques de centre C et, où la tête de massage comprend un mécanisme de transmission configuré pour être entraîné par les moyens de motorisation et pour rapprocher les embouts de massage et/ou, inversement, pour écarter les embouts de massage, en translatant lesdits embouts de massage dans un sens et/ou dans l'autre selon des trajectoires (T₁, T₂) qui se rejoignent au centre C.

2. Appareil de massage (1) selon la revendication 1, lequel comprend trois embouts de massage (123, 124, 125), le mécanisme de transmission étant configuré pour translater concomitamment les embouts de massage dans un sens puis, inversement, dans l'autre sens, les trajectoires (T₂) étant radiales.

3. Appareil de massage (1) selon la revendication 2, dans lequel le mécanisme de transmission comprend :
- un système de guidage (116) fixe sur la tête de massage (4), trois liaisons glissières (117, 118, 119) étant agencées respectivement entre les trois embouts de massage (123, 124, 125) et le système de guidage et configurées pour translater radialement vis-à-vis du centre C lesdits embouts de massage,
- un système de retour (164) des embouts de massage configuré pour exercer une poussée contre les embouts de massage visant à écarter lesdits embouts de massage en translation sur le système de guidage et,
- un système de rapprochement des embouts de massage (120, 121, 122, 164) configuré pour être entraîné en rotation par les moyens de motorisation selon un axe (X₁) perpendiculaire à la surface d'application et passant par le centre C et, d'une part, pour pousser contre les embouts de massage et les rapprocher en translation sur le système de guidage, sur des premières portions angulaires durant la rotation du système de rapprochement des embouts de massage et, d'autre part, pour autoriser le système de retour des embouts de massage à écarter les embouts de massage en translation sur le système de guidage, sur des secondes portions angulaires durant la rotation du système de rapprochement des embouts de massage.

4. Appareil de massage (1) selon la revendication 1, lequel comprend trois embouts de massage (21, 22, 23), le mécanisme de transmission étant configuré pour translater concomitamment les embouts de massage dans un sens puis, inversement, dans l'autre sens, les trajectoires (T₁) étant curvilignes.

5. Appareil de massage (1) selon la revendication 4, dans lequel le mécanisme de transmission comprend :
- un système de guidage (10) configuré pour être entraîné en rotation par les moyens de motorisation selon un axe (X₁) perpendiculaire à la surface d'application (20) et passant par le centre C, trois liaisons glissières (33, 34, 35) étant agencées respectivement entre les trois embouts de massage (21, 22, 23) et le système de guidage et configurées pour translater radialement vis-à-vis du centre C lesdits embouts de massage,
- un système de retour des embouts de massage (70, 164) configuré pour exercer une poussée contre les embouts de massage visant à écarter lesdits embouts de massage en translation sur le système de guidage et,
- un système de rapprochement des embouts de massage (19, 164) fixe sur la tête de massage (4) et configuré, d'une part, pour pousser contre les embouts de massage et les rapprocher en translation sur le système de guidage, sur des premières portions angulaires durant la rotation dudit système de guidage et, d'autre part, pour autoriser le système de retour des embouts de massage à écarter les embouts de massage en translation sur le système de guidage, sur des secondes portions angulaires durant la rotation dudit système de guidage.

6. Appareil de massage (1) selon la revendication 1, lequel comprend au moins trois embouts de massage (128, 129, 130), le mécanisme de transmission étant configuré pour translater avec un décalage régulier les embouts de massage dans un sens puis, inversement, dans l'autre sens, les trajectoires (T₁) étant radiales.

7. Appareil de massage (1) selon la revendication 6, dans lequel le mécanisme de transmission comprend :
- un système de guidage (116) fixe sur la tête de massage, trois liaisons glissières (117, 118, 119) étant agencées respectivement entre les trois embouts de massage (128, 129, 130) et le système de guidage et configurées pour translater radialement vis-à-vis du centre C lesdits embouts de massage,
- un système de retour des embouts de massage configuré pour exercer une poussée contre les embouts de massage visant à écarter lesdits embouts de massage en translation sur le système de guidage et,
- un système de rapprochement des embouts de massage (126) configuré pour être entraîné en rotation par les moyens de motorisation (3) selon un axe (X₁) perpendiculaire à la surface d'application et passant par le centre (C) et, pour pousser progressivement contre un des trois embouts de massage (129) et le rapprocher en translation sur le système de guidage et concomitamment, pour autoriser le système de retour des embouts de massage à écarter progressivement l'embout de massage précédant (128), en translation sur le système de guidage, durant la rotation du système de rapprochement des embouts de massage, et ainsi de suite successivement sur les trois embouts durant la rotation dudit système de rapprochement des embouts de massage.

8. Appareil de massage (1) selon la revendication 1, lequel comprend quatre embouts de massage (81, 82, 83, 84), le mécanisme de transmission étant configuré pour translater concomitamment les embouts de massage dans un sens puis, inversement, dans l'autre sens, les trajectoires (T₁) étant radiales.

9. Appareil de massage (1) selon la revendication 8, dans lequel le mécanisme de transmission comprend :
- un système de guidage (75) fixe sur la tête de massage, quatre liaisons glissières (85, 86, 87, 88) étant agencées respectivement entre les quatre embouts de massage (81, 82, 83, 84) et le système de guidage et configurées pour translater radialement vis-à-vis du centre C lesdits embouts de massage,
- un système de retour des embouts de massage (103, 164) configuré pour exercer une poussée contre les embouts de massage visant à écarter lesdits embouts de massage en translation sur le système de guidage et,
- un système de rapprochement des embouts de massage (104, 108, 109, 110, 111, 164) configuré pour être entraîné en rotation par les moyens de motorisation (3) selon un axe (X₁) perpendiculaire à la surface d'application et passant par le centre C et, d'une part, pour pousser contre les embouts de massage et les rapprocher en translation sur le système de guidage, sur des premières portions angulaires durant la rotation du système de rapprochement des embouts de massage et, d'autre part, pour autoriser le système de retour des embouts de massage à écarter les embouts de massage en translation sur le système de guidage, sur des secondes portions angulaires durant la rotation du système de rapprochement des embouts de massage.

10. Appareil de massage (1) selon la revendication 1, lequel comprend quatre embouts de massage, le mécanisme de transmission étant configuré pour translater concomitamment les embouts de massage dans un sens puis, inversement, dans l'autre sens, les trajectoires (T₂) étant curvilignes.

11. Appareil de massage (1) selon la revendication 10, dans lequel le mécanisme de transmission comprend :
- un système de guidage configuré pour être entraîné en rotation par les moyens de motorisation (3) selon un axe (X₁) perpendiculaire à la surface d'application et passant par le centre C, quatre liaisons glissières étant agencées respectivement entre les quatre embouts de massage et le système de guidage et configurées pour translater radialement vis-à-vis du centre C lesdits embouts de massage,
- un système de retour des embouts de massage configuré pour exercer une poussée contre les embouts de massage visant à écarter lesdits embouts de massage en translation sur le système de guidage et,
- un système de rapprochement des embouts de massage fixe sur la tête de massage (4) et configuré, d'une part, pour pousser contre les embouts de massage et les rapprocher en translation sur le système de guidage, sur des premières portions angulaires durant la rotation dudit système de guidage et, d'autre part, pour autoriser le système de retour des embouts de massage à écarter les embouts de massage en translation sur le système de guidage, sur des secondes portions angulaires durant la rotation dudit système de guidage.

12. Appareil de massage (1) selon la revendication 1, lequel comprend quatre embouts de massage (81, 82, 83, 84), le mécanisme de transmission étant configuré pour translater concomitamment dans un sens, les deux premiers embouts de massage (81, 83) disposés en vis-à-vis et dans l'autre sens, les deux seconds embouts de massage (82, 84) disposés en vis-à-vis, puis inversement, les trajectoires (T₁) étant radiales.

13. Appareil de massage (1) selon la revendication 12, dans lequel le mécanisme de transmission comprend :
- un système de guidage (75) fixe sur la tête de massage (4), quatre liaisons glissières (85, 86, 87, 88) étant agencées respectivement entre les quatre embouts de massage (81, 82, 83, 84) et le système de guidage et configurées pour translater radialement vis-à-vis du centre C lesdits embouts de massage,
- un système de retour des embouts de massage (103) configuré pour exercer une poussée contre les embouts de massage visant à écarter lesdits embouts de massage en translation sur le système de guidage et,
- un système de rapprochement des embouts de massage (133, 134) configuré pour être entraîné en rotation par les moyens de motorisation selon un axe (X₁) perpendiculaire à la surface d'application et passant par le centre C et, pour pousser contre les deux premiers embouts de massage (81, 83) en vis-à-vis et les rapprocher en translation sur le système de guidage et, concomitamment, pour autoriser le système de retour des embouts de massage à écarter en translation sur le système de guidage les deux seconds embouts de massage (82, 84) en vis-à-vis, sur des premières portions angulaires durant la rotation du système de rapprochement des embouts de massage et, inversement sur des secondes portions angulaires durant la rotation du système de rapprochement des embouts de massage.

14. Appareil de massage (1) selon l'une des revendications 3, 5, 7, 9, 11 ou 13, dans lequel le système de rapprochement des embouts de massage comprend des moyens d'amortissement (70, 103, 175) des efforts exercés par les embouts de massage sur la peau.

15. Appareil de massage (1) selon l'une des revendications 1 à 14, lequel comprend une couronne (189) s'étendant perpendiculairement à la surface d'application et, un second mécanisme de transmission (194) entraîné par les moyens de motorisation (3), configuré pour entraîner la couronne avec un mouvement oscillatoire ou de rotation autour d'un axe (X₁) perpendiculaire à la surface d'application (190) et passant par le centre (C).

16. Appareil de massage (1) selon l'une des revendications 1 à 15, lequel comprend un système d'émission d'ondes (186) au niveau de la surface d'application (6).

17. Appareil de massage (1) selon l'une des revendications 1 à 16, dans lequel les embouts de massage (179) sont dans une matière et de forme configurées pour accrocher et travailler la peau sans douleur.

18. Appareil de massage (1) selon l'une des revendications 1 à 17, lequel comprend un système de distribution (204) d'un produit cosmétique configuré pour distribuer ledit produit cosmétique sur les embouts de massage (213) et/ou directement sur la peau.

19. Appareil de massage (1) selon l'une des revendications 1 à 18, lequel comprend une peau souple (180) qui recouvre les embouts de massage (183, 184, 185).

20. Appareil de massage (1) selon l'une des revendications 1 à 19, lequel comprend un système vibratoire (36) configuré pour faire vibrer la tête de massage ou les embouts de massage(4).

21. Appareil de massage (1) selon l'une des revendications 1 à 20, dans lequel les moyens d'assemblage entre le corps (2) et la tête de massage (4) sont configurés pour connecter de manière amovible la tête de massage sur le corps.

22. Appareil de massage (1) selon l'une des revendications 1 à 21, lequel comprend un dispositif de traitement ionophorèse transcutanée (221, 222, 224) qui est configuré pour transmettre sur la peau lors de l'application dudit appareil de massage, un courant permettant d'augmenter et/ou d'accélérer la pénétration d'un produit cosmétique.

## Patentansprüche

1. Massagegerät (1) mit einem Körper (2), der Antriebsmittel (3, 4) und einen Massagekopf (5) aufweist, wobei zwischen dem Körper und dem Massagekopf Montagemittel angeordnet sind, wobei der genannte Massagekopf eine Applikationsfläche und zumindest drei Massagestifte aufweist, die sich auf der Applikationsfläche nach außen erstrecken, wobei die Massagestifte auf der Applikationsfläche in virtuellen konzentrischen Kreisen mit dem Mittelpunkt C definiert sind und der Massagekopf einen Übertragungsmechanismus umfasst, der so konfiguriert ist, dass er von den Antriebsmitteln angetrieben wird, um die Massagestifte zueinander hin und/oder entgegengesetzt voneinander weg zu bewegen, indem die genannten Massagestifte in der einen und/oder der anderen Richtung entlang der Bahnen (T₁, T₂), die sich im Mittelpunkt C treffen, bewegt werden.

2. Massagegerät (1) nach Anspruch 1, das drei Massagestifte (123, 124, 125) umfasst, wobei der Übertragungsmechanismus so konfiguriert ist, dass er die Massagestifte gleichzeitig in die eine Richtung und dann entgegengesetzt in die andere Richtung bewegt, wobei die Bahnen (T₂) radial verlaufen.

3. Massagegerät (1) nach Anspruch 2, wobei der Übertragungsmechanismus Folgendes umfasst:
- ein ortsfestes Führungssystem (116) auf dem Massagekopf (4), drei Gleitverbindungen (117, 118, 119), die jeweils zwischen den drei Massagestiften (123, 124, 125) und dem Führungssystem angeordnet und so konfiguriert sind, dass sie die genannten Massagestifte radial in Bezug auf den Mittelpunkt C bewegen,
- ein System (164) zur Rückführung der Massagestifte, das so konfiguriert ist, dass es gegen die Massagestifte einen Druck ausübt, der darauf abzielt, die genannten Massagestifte auf dem Führungssystem voneinander weg zu bewegen, und
- ein System zur Annäherung der Massagestifte (120, 121, 122, 164), das so konfiguriert ist, dass es von den Antriebsmitteln um eine Achse (X₁), die senkrecht zur Applikationsfläche steht und durch den Mittelpunkt C verläuft, in Drehung versetzt wird, um einerseits in den ersten Winkelabschnitten während der Drehung des Systems zur Annäherung der Massagestifte gegen die Massagestifte zu drücken und sie auf dem Führungssystem zueinander hin zu bewegen und andererseits in den zweiten Winkelabschnitten während der Drehung des Systems zur Annäherung der Massagestifte dem System zur Rückführung der Massagestifte zu gestatten, die Massagestifte auf dem Führungssystem voneinander weg zu bewegen.

4. Massagegerät (1) nach Anspruch 1, das drei Massagestifte (21, 22, 23) umfasst, wobei der Übertragungsmechanismus so konfiguriert ist, dass er die Massagestifte gleichzeitig in die eine Richtung und dann entgegensetzt in die andere Richtung bewegt, wobei die Bahnen (T₁) krummlinig verlaufen.

5. Massagegerät (1) nach Anspruch 4, wobei der Übertragungsmechanismus Folgendes umfasst:
- ein Führungssystem (10), das so konfiguriert ist, dass es von den Antriebsmitteln um eine Achse (X₁), die senkrecht zur Applikationsfläche (20) steht und durch den Mittelpunkt C verläuft, in Drehung versetzt wird, wobei drei Gleitverbindungen (33, 34, 35) jeweils zwischen den drei Massagestiften (21, 22, 23) und dem Führungssystem angeordnet und so konfiguriert sind, dass sie die genannten Massagestifte radial in Bezug auf den Mittelpunkt C bewegen,
- ein System (70, 164) zur Rückführung der Massagestifte, das so konfiguriert ist, dass es gegen die Massagestifte einen Druck ausübt, der darauf abzielt, die genannten Massagestifte auf dem Führungssystem voneinander weg zu bewegen, und
- ein System zur Annäherung der Massagestifte (19, 164), das ortsfest auf dem Massagekopf (4) und so konfiguriert ist, dass es einerseits in den ersten Winkelabschnitten während der Drehung des genannten Führungssystems gegen die Massagestifte drückt und sie auf dem Führungssystem zueinander hin bewegt und andererseits in den zweiten Winkelabschnitten während der Drehung des genannten Führungssystems dem System zur Rückführung der Massagestifte gestattet, die Massagestifte auf dem Führungssystem voneinander weg zu bewegen.

6. Massagegerät (1) nach Anspruch 1, das zumindest drei Massagestifte (128, 129, 130) umfasst, wobei der Übertragungsmechanismus so konfiguriert ist, dass er die Massagestifte mit einem regelmäßigen Versatz in die eine Richtung und dann entgegengesetzt in die andere Richtung bewegt, wobei die Bahnen (T₁) radial verlaufen.

7. Massagegerät (1) nach Anspruch 6, wobei der Übertragungsmechanismus Folgendes umfasst:
- ein ortsfestes Führungssystem (116) auf dem Massagekopf, drei Gleitverbindungen (117, 118, 119), die jeweils zwischen den drei Massagestiften (128, 129, 130) und dem Führungssystem angeordnet und so konfiguriert sind, dass sie die genannten Massagestifte radial in Bezug auf den Mittelpunkt C bewegen,
- ein System zur Rückführung der Massagestifte, das so konfiguriert ist, dass es gegen die Massagestifte einen Druck ausübt, der darauf abzielt, die genannten Massagestifte auf dem Führungssystem voneinander weg zu bewegen, und
- ein System zur Annäherung der Massagestifte (126), das so konfiguriert ist, dass es von den Antriebsmitteln (3) um eine Achse (X₁), die senkrecht zur Applikationsfläche steht und durch den Mittelpunkt C verläuft, in Drehung versetzt wird, um zunehmend gegen einen der drei Massagestifte (129) zu drücken und ihn auf dem Führungssystem hin zu bewegen und gleichzeitig während der Drehung des Systems zur Annäherung der Massagestifte dem System zur Rückführung der Massagestifte zu gestatten, den vorhergehenden Massagestift (128) auf dem Führungssystem zunehmend weg zu bewegen, und so fort nacheinander bei den drei Stiften während der Drehung des genannten Systems zur Annäherung der Massagestifte.

8. Massagegerät (1) nach Anspruch 1, das vier Massagestifte (81, 82, 83, 84) umfasst, wobei der Übertragungsmechanismus so konfiguriert ist, dass er die Massagestifte gleichzeitig in die eine Richtung und dann entgegensetzt in die andere Richtung bewegt, wobei die Bahnen (T₁) radial verlaufen.

9. Massagegerät (1) nach Anspruch 8, wobei der Übertragungsmechanismus Folgendes umfasst:
- ein ortsfestes Führungssystem (75) auf dem Massagekopf, vier Gleitverbindungen (85, 86, 87, 88), die jeweils zwischen den vier Massagestiften (81, 82, 83, 84) und dem Führungssystem angeordnet und so konfiguriert sind, dass sie die genannten Massagestifte radial in Bezug auf den Mittelpunkt C bewegen,
- ein System zur Rückführung der Massagestifte (103, 164), das so konfiguriert ist, dass es gegen die Massagestifte einen Druck ausübt, der darauf abzielt, die genannten Massagestifte auf dem Führungssystem voneinander weg zu bewegen, und
- ein System zur Annäherung der Massagestifte (104, 108, 109, 110, 111, 164), das so konfiguriert ist, dass es von den Antriebsmitteln (3) um eine Achse (X₁), die senkrecht zur Applikationsfläche steht und durch den Mittelpunkt C verläuft, in Drehung versetzt wird, um einerseits in den ersten Winkelabschnitten während der Drehung des Systems zur Annäherung der Massagestifte gegen die Massagestifte zu drücken und sie auf dem Führungssystem zueinander hin zu bewegen und andererseits in den zweiten Winkelabschnitten während der Drehung des Systems zur Annäherung der Massagestifte dem System zur Rückführung der Massagestifte zu gestatten, die Massagestifte auf dem Führungssystem voneinander weg zu bewegen.

10. Massagegerät (1) nach Anspruch 1, das vier Massagestifte umfasst, wobei der Übertragungsmechanismus so konfiguriert ist, dass er die Massagestifte gleichzeitig in die eine Richtung und dann entgegensetzt in die andere Richtung bewegt, wobei die Bahnen (T₂) krummlinig verlaufen.

11. Massagegerät (1) nach Anspruch 10, wobei der Übertragungsmechanismus Folgendes umfasst:
- ein Führungssystem, das so konfiguriert ist, dass es von den Antriebsmitteln (3) um eine Achse (X₁), die senkrecht zur Applikationsfläche steht und durch den Mittelpunkt C verläuft, in Drehung versetzt wird, wobei vier Gleitverbindungen jeweils zwischen den vier Massagestiften und dem Führungssystem angeordnet und so konfiguriert sind, dass sie die genannten Massagestifte radial in Bezug auf den Mittelpunkt C bewegen,
- ein System zur Rückführung der Massagestifte, das so konfiguriert ist, dass es gegen die Massagestifte einen Druck ausübt, der darauf abzielt, die genannten Massagestifte auf dem Führungssystem voneinander weg zu bewegen, und
- ein System zur Annäherung der Massagestifte, das ortsfest auf dem Massagekopf (4) und so konfiguriert ist, dass es einerseits in den ersten Winkelabschnitten während der Drehung des genannten Führungssystems gegen die Massagestifte drückt und sie auf dem Führungssystem zueinander hin bewegt und andererseits in den zweiten Winkelabschnitten während der Drehung des genannten Führungssystems dem System zur Rückführung der Massagestifte gestattet, die Massagestifte auf dem Führungssystem voneinander weg zu bewegen.

12. Massagegerät (1) nach Anspruch 1, das vier Massagestifte (81, 82, 83, 84) umfasst, wobei der Übertragungsmechanismus so konfiguriert ist, dass er die beiden ersten, sich gegenüber liegenden Massagestifte (81, 83) gleichzeitig in die eine Richtung und die beiden zweiten, sich gegenüber liegenden Massagestifte (82, 84) in die andere Richtung bewegt, und anschließend umgekehrt, wobei die Bahnen (T₁) radial verlaufen.

13. Massagegerät (1) nach Anspruch 12, wobei der Übertragungsmechanismus Folgendes umfasst:
- ein ortsfestes Führungssystem (75) auf dem Massagekopf (4), vier Gleitverbindungen (85, 86, 87, 88), die jeweils zwischen den vier Massagestiften (81, 82, 83, 84) und dem Führungssystem angeordnet und so konfiguriert sind, dass sie die genannten Massagestifte radial in Bezug auf den Mittelpunkt C bewegen,
- ein System zur Rückführung der Massagestifte (103), das so konfiguriert ist, dass es gegen die Massagestifte einen Druck ausübt, der darauf abzielt, die genannten Massagestifte auf dem Führungssystem voneinander weg zu bewegen, und
- ein System zur Annäherung der Massagestifte (133, 134), das so konfiguriert ist, dass es von den Antriebsmitteln um eine Achse (X₁), die senkrecht zur Applikationsfläche steht und durch den Mittelpunkt C verläuft, in Drehung versetzt wird, um in den ersten Winkelabschnitten während der Drehung des Systems zur Annäherung der Massagestifte gegen die beiden ersten, sich gegenüber liegenden Massagestifte (81, 83) zu drücken und sie auf dem Führungssystem zueinander hin zu bewegen, und gleichzeitig dem System zur Rückführung der Massagestifte zu gestatten, die beiden zweiten, sich gegenüber liegenden Massagestifte (82, 84) auf dem Führungssystem voneinander weg zu bewegen, und in den zweiten Winkelabschnitten während der Drehung des Systems zur Annäherung der Massagestifte umgekehrt.

14. Massagegerät (1) nach einem der Ansprüche 3, 5, 7, 9, 11 oder 13, bei dem das System zur Annäherung der Massagestifte Mittel (70, 103, 175) zur Dämpfung der von den Massagestiften auf die Haut ausgeübten Kräfte umfasst.

15. Massagegerät (1) nach einem der Ansprüche 1 bis 14, das einen Kranz (189), der sich senkrecht zur Applikationsfläche erstreckt, und einen zweiten Übertragungsmechanismus (194) umfasst, der von den Antriebsmitteln (3) angetrieben wird und so konfiguriert ist, dass er den Kranz mit einer Schwing- oder Drehbewegung um eine Achse (X₁), die senkrecht zur Applikationsfläche (190) steht und durch den Mittelpunkt (C) verläuft, antreibt.

16. Massagegerät (1) nach einem der Ansprüche 1 bis 15, das im Bereich der Applikationsfläche (6) ein System (186) zur Aussendung von Wellen umfasst.

17. Massagegerät (1) nach einem der Ansprüche 1 bis 16, bei dem die Massagestifte (179) aus einem Material bestehen und eine Form aufweisen, die so ausgelegt sind, dass sie die Haut schmerzfrei greifen und bearbeiten.

18. Massagegerät (1) nach einem der Ansprüche 1 bis 17, das ein System (204) zur Verteilung eines Kosmetikprodukts umfasst, das so konfiguriert ist, dass es das genannte Kosmetikprodukt auf den Massagestiften (213) und/oder direkt auf der Haut verteilt.

19. Massagegerät (1) nach einem der Ansprüche 1 bis 18, das eine weiche Haut (180) umfasst, die die Massagestifte (183, 184, 185) bedeckt.

20. Massagegerät (1) nach einem der Ansprüche 1 bis 19, das ein Vibrationssystem (36) umfasst, das so konfiguriert ist, dass es den Massagekopf oder die Massagestifte (4) vibrieren lässt.

21. Massagegerät (1) nach einem der Ansprüche 1 bis 20, bei dem die Montagemittel zwischen dem Körper (2) und dem Massagekopf (4) so konfiguriert sind, dass sie den Massagekopf abnehmbar mit dem Körper verbinden.

22. Massagegerät (1) nach einem der Ansprüche 1 bis 21, das eine Vorrichtung zur transkutanen Behandlung durch lontophorese (221, 222, 224) umfasst, die dafür ausgelegt ist, bei Anwendung des genannten Massagegerätes einen Strom auf die Haut zu leiten, durch den sich das Eindringen eines Kosmetikprodukts erhöhen und/oder beschleunigen lässt.

## Claims

1. Massage apparatus (1) comprising a body (2) having drive means (3, 4) and a massage head (5), assembly means being arranged between the body and the massage head, said massage head comprising an application surface and at least three massaging tips which extend towards the outside of the application surface, wherein the massaging tips are defined on the application surface according to concentric virtual circles of centre C and wherein the massage head comprises a transmission mechanism configured to be driven by the drive means and to bring closer the massaging tips and/or, conversely, to move away the massaging tips, by translating said massaging tips in one direction and/or in the other direction according to the trajectories (T₁, T₂) which meet at the centre C.

2. Massage apparatus (1) according to claim 1, which comprises three massaging tips (123, 124, 125), the transmission mechanism being configured to translate simultaneously the massaging tips in one direction then, conversely, in the other direction, the trajectories (T₂) being radial.

3. Massage apparatus (1) according to claim 2, wherein the transmission mechanism comprises:
- a fixed guide system (116) on the massage head (4), three slide links (117, 118, 119) being arranged respectively between the three massaging tips (123, 124, 125) and the guide system and configured to translate said massaging tips radially with respect to the centre C,
- a system (164) for returning the massaging tips configured to exert a thrust against the massaging tips to move away said massaging tips in translation on the guide system, and
- a system for bringing closer the massaging tips (120, 121, 122, 164) configured to be driven in rotation by the drive means about an axis (X₁) perpendicular to the application surface and passing through the centre C and, firstly, to push against the massaging tips and bring them closer in translation on the guide system, on first angular portions during rotation of the system for bringing closer the massaging tips and, secondly, to allow the system for returning the massaging tips to move away the massaging tips in translation on the guide system, on second angular portions during rotation of the system for bringing closer the massaging tips.

4. Massage apparatus (1) according to claim 1, which comprises three massaging tips (21, 22, 23), the transmission mechanism being configured to translate simultaneously the massaging tips in one direction then, conversely, in the other direction, the trajectories (T₁) being curved.

5. Massage apparatus (1) according to claim 4, wherein the transmission mechanism comprises:
- a guide system (10) configured to be driven in rotation by the drive means about an axis (X₁) perpendicular to the application surface (20) and passing through the centre C, three slide links (33, 34, 35) being arranged respectively between the three massaging tips (21, 22, 23) and the guide system and configured to translate said massaging tips radially with respect the centre C,
- a system for returning the massaging tips (70, 164) configured to exert a thrust against the massaging tips to move away said massaging tips in translation on the guide system, and
- a fixed system for bringing closer the massaging tips (19, 164) on the massage head (4) and configured, firstly, to push against the massaging tips and bring them closer in translation on the guide system, on first angular portions during rotation of said guide system and, secondly, to allow the system for returning the massaging tips to move away the massaging tips in translation on the guide system, on second angular portions during rotation of said guide system.

6. Massage apparatus (1) according to claim 1, which comprises at least three massaging tips (128, 129, 130), the transmission mechanism being configured to translate the massaging tips with a regular shift in one direction then, conversely, in the other direction, the trajectories (T₁) being radial.

7. Massage apparatus (1) according to claim 6, wherein the transmission mechanism comprises:
- a fixed guide system (116) on the massage head, three slide links (117, 118, 119) being arranged respectively between the three massaging tips (128, 129, 130) and the guide system and configured to translate said massaging tips radially with respect to the centre C,
- a system for returning the massaging tips configured to exert a thrust against the massaging tips to move away said massaging tips in translation on the guide system, and
- a system for bringing closer the massaging tips (126) configured to be driven in rotation by the drive means (3) about an axis (X₁) perpendicular to the application surface and passing through the centre (C) and, to progressively push against one of the three massaging tips (129) and bring it closer in translation on the guide system, and simultaneously to allow the system for returning the massaging tips to progressively move away the preceding massaging tip (128), in translation on the guide system, during rotation of the system for bringing closer the massaging tips and so on successively on the three tips during rotation of said system for bringing closer the massaging tips.

8. Massage apparatus (1) according to claim 1, which comprises four massaging tips (81, 82, 83, 84), the transmission mechanism being configured to translate simultaneously the massaging tips in one direction then, conversely, in the other direction, the trajectories (T₁) being radial.

9. Massage apparatus (1) according to claim 8, wherein the transmission mechanism comprises:
- a fixed guide system (75) on the massage head, four slide links (85, 86, 87, 88) being arranged respectively between the four massaging tips (81, 82, 83, 84) and the guide system and configured to translate said massaging tips radially with respect to the centre C,
- a system for returning the massaging tips (103, 164) configured to exert a thrust against the massaging tips to move away said massaging tips in translation on the guide system, and
- a system for bringing closer the massaging tips (104, 108, 109, 110, 111, 164) configured to be driven in rotation by the drive means (3) about an axis (X₁) perpendicular to the application surface and passing through the centre C and, firstly, to push against the massaging tips and bring them closer in translation on the guide system, on first angular portions during rotation of the system for bringing closer the massaging tips and, secondly, to allow the system for returning the massaging tips to move away the massaging tips in translation on the guide system, on second angular portions during rotation of the system for bringing closer the massaging tips.

10. Massage apparatus (1) according to claim 1, which comprises four massaging tips, the transmission mechanism being configured to translate simultaneously the massaging tips in one direction then, conversely, in the other direction, the trajectories (T₂) being curved.

11. Massage apparatus (1) according to claim 10, wherein the transmission mechanism comprises:
- a guide system configured to be driven in rotation by the drive means (3) about an axis (X₁) perpendicular to the application surface (20) and passing through the centre C, four slide links being arranged respectively between the four massaging tips and the guide system and configured to translate said massaging tips radially with respect to the centre C,
- a system for returning the massaging tips configured to exert a thrust against the massaging tips to move away said massaging tips in translation on the guide system, and
- a fixed system for bringing closer the massaging tips on the massage head (4) and configured, firstly, to push against the massaging tips and bring them closer in translation on the guide system, on first angular portions during rotation of said guide system and, secondly, to allow the system for returning the massaging tips to move away the massaging tips in translation on the guide system, on second angular portions during rotation of said guide system.

12. Massage apparatus (1) according to claim 1, which comprises four massaging tips (81, 82, 83, 84), the transmission mechanism being configured to translate simultaneously in one direction, the first two massaging tips (81, 83) arranged opposite each other and in the other direction, the second two massaging tips (82, 84) arranged opposite each other, then conversely, the trajectories (T₁) being radial.

13. Massage apparatus (1) according to claim 12, wherein the transmission mechanism comprises:
- a fixed guide system (75) on the massage head (4), four slide links (85, 86, 87, 88) being arranged respectively between the four massaging tips (81, 82, 83, 84) and the guide system and configured to translate said massaging tips radially with respect to the centre C,
- a system for returning the massaging tips (103) configured to exert a thrust against the massaging tips to move away said massaging tips in translation on the guide system, and
- a system for bringing closer the massaging tips (133, 134) configured to be driven in rotation by the drive means about an axis (X₁) perpendicular to the application surface and passing through the centre C and to push against the first two massaging tips (81, 83) arranged opposite each other and bring them closer in translation on the guide system and, simultaneously, to allow the system for returning the massaging tips to move away the second two massaging tips (82, 84) arranged opposite each other in translation on the guide system, on first angular portions during rotation of the system for bringing closer the massaging tips and, conversely, on second angular portions during rotation of the system for bringing closer the massaging tips.

14. Massage apparatus (1) according to one of claims 3, 5, 7, 9, 11 or 13, wherein the system for bringing closer the massaging tips comprises means (70, 103, 175) for damping the forces exerted by the massaging tips on the skin.

15. Massage apparatus (1) according to one of claims 1 to 14, which comprises a ring gear (189) extending perpendicular to the application surface, and a second transmission mechanism (194) driven by the drive means (3) configured to drive the ring gear with an oscillating or rotating movement about an axis (X₁) perpendicular to the application surface (190) and passing through the centre (C).

16. Massage apparatus (1) according to one of claims 1 to 15, which comprises a system (186) for transmitting waves onto the application surface (6).

17. Massage apparatus (1) according to one of claims 1 to 16, wherein the material and shape of the massaging tips (179) are configured to adhere to the skin and massage it painlessly.

18. Massage apparatus (1) according to one of claims 1 to 17, which comprises a system (204) for dispensing a cosmetic product configured to dispense said cosmetic product on the massaging tips (213) and/or directly on the skin.

19. Massage apparatus (1) according to one of claims 1 to 18, which comprises a flexible skin (180) covering the massaging tips (183, 184, 185).

20. Massage apparatus (1) according to one of claims 1 to 19, which comprises a vibratory system (36) configured to vibrate the massage head or the massaging tips (4).

21. Massage apparatus (1) according to one of claims 1 to 20, wherein the assembly means between the body (2) and the massage head (4) are configured to removably connect the massage head on the body.

22. Massage apparatus (1) according to one of claims 1 to 21, which comprises a transcutaneous ionophoresis treatment device (221, 222, 224) which is configured to transmit to the skin during application of said massage apparatus, a current used to increase and/or accelerate penetration of a cosmetic product.
